# EUROPEAN PATENT APPLICATION

(11) **EP 1 619 648 A1**
(43) Date of publication of application: **25.01.2006**
(21) Application number: 04723841.5
(22) Date of filing: 26.03.2004
(51) Int. Cl.: G09F 9/00, G09F 9/30, A61M 21/00, H05B 33/12, G09G 3/20, G09G 3/30, G09G 3/34, G09G 5/00, G09G 5/10

(54) **DISPLAY DEVICE**

(30) Priority: 28.03.2003 JP 2003092542
(71) Applicant: SHARP KABUSHIKI KAISHA, Osaka-shi, Osaka 545-8522 (JP)
(72) Inventor: WAKABAYASHI, Yasutaka, (JP); YAMANAKA, Atsushi, (JP); IWAUCHI, Kenichi, (JP); KOYAMA, Emi, (JP)
(74) Representative: Brown, Kenneth Richard
(86) International application number: PCT/JP2004/004364
(87) International publication number: WO 2004/088616

(57) **Abstract**

A display device (1) of the present invention displays an image by using an organic layer (9) which emits light having such a wavelength that affects a biorhythm, and the display device (1) controls a luminous intensity of the organic layer (9).

## Description

### TECHNICAL FIELD

The present invention relates to a display device designed in consideration of an effect of light on a biorhythm.

### BACKGROUND ART

A living organism has in its body a clock mechanism which is known to regulate periodic phenomena regarding vital functions. Known as one of the periodic phenomena is the circadian rhythm, i.e., a rhythm that occurs over a 24-to-25-hour cycle ("A chronobiological understanding of characteristics of a biological system through modeling", *Journal of the Society of Instrument and Control Engineering,* Vol. 41, No. 10, October 2002). Typical examples of the circadian rhythm are sleeping, awakening, body temperature change. Examples of main factors which synchronize each of these rhythms with a 24-hour or one-day cycle are social factors and light environments.

In the modern world, people spend more time indoors, and more of them stay up late thanks to the development of lighting, resulting in a light environment where there is no big difference in brightness between day and night. This may put a biorhythm out of synchronization with a 24-hour cycle, thereby causing health problems such as sleep disorders. For healthy and comfortable living, an organism's biorhythm needs to be in phase with time in the organism's surrounding environment, and large amplitude needs to be ensured. Various methods have been proposed to regulate a biorhythm.

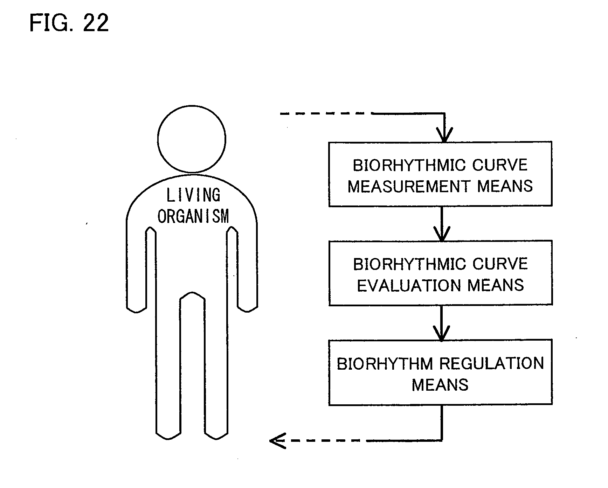

For example, a "biorhythm regulation device" disclosed in Japanese Unexamined Patent Publication No. 3920/1993 (*Tokukaihei* 5-3920; published on January 14, 1993) regulates a subject's biorhythm by measuring and evaluating the subject's biorhythm based on the subject's deep body temperature such as the subject's rectal temperature and by accordingly giving the subject a stimulus such as light. Fig. 22 shows a concrete arrangement of the biorhythm regulation device. As shown in Fig. 22, the biorhythm regulation device is arranged so as to include: biorhythmic curve measurement means for measuring an organism's biorhythm; biorhythmic curve evaluation means for evaluating the organism's biorhythm in comparison with an ideal biorhythm; and a biorhythm regulation device for regulating the organism's biorhythm.

Further, a "lighting control method and a lighting system" disclosed in Japanese Unexamined Patent Publication No. 294387/2000 *(Tokukai* 2000-294387; published on October 20, 2000) include a light having a low color temperature and a light having a high color temperature. The lights are switched or simultaneously turned on in accordance with a period of time such as day or night. Furthermore, an "awakening device" disclosed in Japanese Unexamined Patent Publication No. 318670/1995 *(Tokukaihei* 7-318670; published on December 8, 1995) includes at least three light-producing means respectively having a low illumination intensity, a middle illumination intensity, and a high illumination intensity. The awakening device produces a gradual increase in illumination intensity as a waking time approaches.

Further, the rhythm of sleeping and awakening is deeply related to melatonin secretion, and melatonin secretion is suppressed during awakening. Furthermore, light has an effect on melatonin secretion. That is, a suppressive effect of light having wavelengths of 440 nm to 600 nm on melatonin was studied to find that light having a wavelength of approximately 470 nm in particular has the highest suppressive effect on melatonin ("Action Spectrum for Melatonin Regulation in Humans: Evidence for a Novel Circadian Photoreceptor", *The Journal of Neuroscience,* August 15, 2001). This finding is employed by a "phototherapeutic instrument" disclosed in Japanese Unexamined Patent Publication No. 302276/1990 *(Tokukaihei* 2-302276; published on December 14, 1990).

Thus, various biorhythm regulation devices have been proposed, but the conventional proposals relate to an indoor lighting device and have given little thought to a display device such as a display. However, because a human being obtains mostly by sight information regarding light, light emitted from the display device also has an effect on the human being's biorhythm.

Particularly, as computers become widespread, people nowadays spend more time working with displays. Moreover, because a user is close to a display while working with such a VDT (Video Display Terminal), the user's eyes receive a large amount of light. This means that the light of the display has a stronger effect on the user's biorhythm than the light of the lighting device.

Further, light emitted from light sources of display devices in general including televisions is different from indirect light such as light emitted from lighting devices, because such light emitted from light sources is viewed directly by a user, and therefore the light has a more profound effect on the user's biorhythm than the indirect light.

Thus, in view of an effect of a display on a biorhythm, the light of the display needs to be controlled.

### DISCLOSURE OF INVENTION

The present invention has been made in view of the foregoing problems and has as an object to provide a display device capable of controlling a biorhythm.

In order to attain the foregoing object, a display device of the present invention is a display device for displaying an image based on light of a light emitter, wherein: the light emitter emits light having such a wavelength that affects a biorhythm, and an intensity of the light having the wavelength is controlled, so that the biorhythm is regulated and the image is displayed.

Further, in order to attain the foregoing object, a display device of the present invention is a display device including an image display section for displaying an image, the image display section including pixels each of which has a plurality of light emitters, wherein: the plurality of light emitters include a first light emitter for emitting light having such a wavelength that affects a biorhythm, and a characteristic of a luminous intensity of the first light emitter with respect to a video signal inputted into the image display section is switched.

Further, in order to attain the foregoing object, a display device of the present invention is a display device irradiating an image display section, which is for displaying an image, with light from a light source so as to cause the image display section to display the image, the display device including: a plurality of light emitters which include a first light emitter for emitting light having such a wavelength that affects a biorhythm, a luminous intensity of the first light emitter with respect to an video signal inputted into the image display section being switched.

According to the foregoing arrangements, by controlling an intensity of light having such a wavelength that affects a biorhythm, the biorhythm can be regulated. Further, by changing a luminous intensity of the first light emitter, an intensity of light having such a wavelength that affects a biorhythm can be controlled. This makes it possible to regulate a biorhythm.

Particularly, because a display device is used in a position closer to a user than is a lighting device, light from the light emitter or the first light emitter is sent directly to the user's eyes, thereby greatly affecting the user's biorhythm. Further, a display device for displaying an image is more frequently used than a lighting device.

Therefore, a luminous intensity of a light emitter of a display device has a more profound effect on the user's biorhythm than does a luminous intensity of a lighting device. According to the present invention, it is possible to effectively regulate the user's biorhythm.

For a fuller understanding of the nature and advantages of the invention, reference should be made to the ensuing detailed description taken in conjunction with the accompanying drawings.

### BRIEF DESCRIPTION OF DRAWINGS

Fig. 1(a) illustrates an arrangement of a display device according to one embodiment of the present invention, and Fig. 1 (b) is a cross-sectional view of an arrangement of each cell of the display device illustrated in Fig. 1(a).
Fig. 2 shows a relationship between a dominant wavelength of a light source and a spectrum locus.
Fig. 3 shows a relationship between a wavelength regarding a biorhythm and an intensity of a suppressive effect on melatonin.
Fig. 4 shows a relationship between an input video signal value of an organic layer 9 and a luminous intensity of the organic layer 9 in the display device of Fig. 1.
Fig. 5 illustrates an arrangement of a liquid crystal display device according to another embodiment of the present invention.
Fig. 6 shows a change on a chromaticity diagram when a color balance of the liquid crystal display device of Fig. 5 is changed.
Fig. 7 shows respective luminous intensities of LEDs when a color temperature of reference white light of the liquid crystal display device of Fig. 5 is set at 5000 K.
Fig. 8 shows respective luminous intensities of LEDs when the color temperature of the reference white light of the liquid crystal display device of Fig. 5 is set at 12000 K.
Fig. 9 shows a relationship between an amount of light of an LED 14 and an input image signal in the liquid crystal display device of Fig. 5.
Fig. 10 illustrates an arrangement of a liquid crystal display device according to a further embodiment of the present invention.
Fig. 11 is a flow chart showing the steps of controlling a luminous intensity of an LED in the liquid crystal display device of Fig. 10.
Fig. 12 illustrates an arrangement of a liquid crystal display device according to a further embodiment of the present invention.
Fig. 13 is a diagram for explaining a complementary wavelength of an LED 14 in the liquid crystal display device of Fig. 12.
Fig. 14 is a graph showing a relationship between a luminous intensity of the LED 14 and a luminous intensity of an LED 32 in the liquid crystal display devices of Figs. 12 and 15.
Fig. 15 illustrates an arrangement of a liquid crystal display device according to a further embodiment of the present invention.
Fig. 16 is a cross-sectional view of an arrangement of a pixel of a light-emitting display device serving as a modification example of the liquid crystal display device of Fig. 15.
Fig. 17 illustrates an arrangement of a liquid crystal display device according to a further embodiment of the present invention.
Fig. 18 illustrates an arrangement of a liquid crystal display device according to a further embodiment of the present invention.
Fig. 19 shows an emission spectrum of a cold cathode fluorescent lamp 22 in the liquid crystal display device of Fig. 18.
Fig. 20 shows a transmission characteristic of a dielectric multilayer filter 72 in the liquid crystal display device of Fig. 18.
Fig. 21 shows a transmission characteristic of a dielectric multilayer filter 73 in the liquid crystal display device of Fig. 18.
Fig. 22 is a diagram for explaining a conventional biorhythm regulation device.

### BEST MODE FOR CARRYING OUT THE INVENTION

### [First Embodiment]

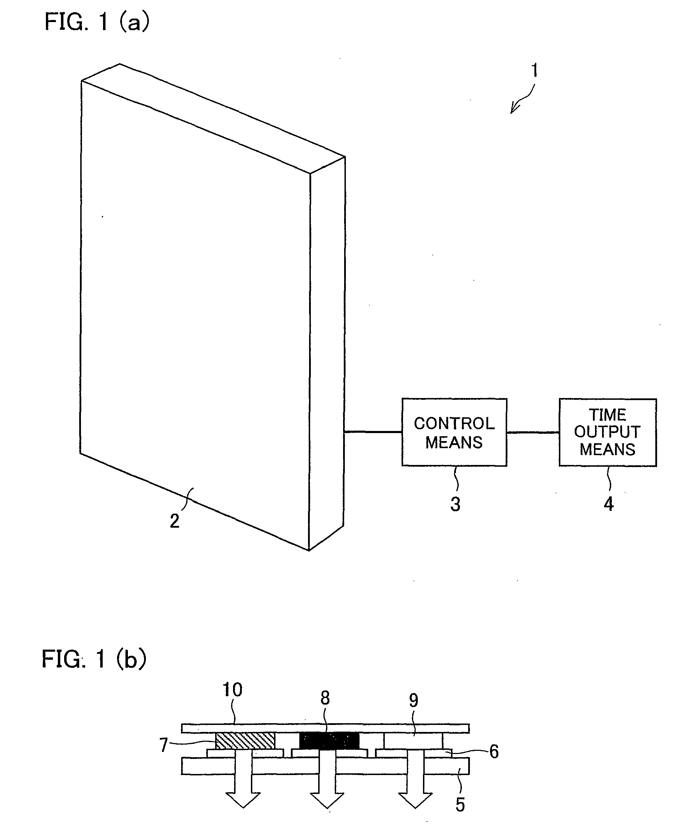

A display device according to one embodiment of the present invention will be described below with reference to Fig. 1. As illustrated in Fig. 1 (a), a display device 1 of the present embodiment includes an organic EL (electroluminescent) panel 2 (image display section) for displaying information such as an image, control means 3 for controlling a current to be applied to each pixel of the organic EL panel 2, and time output means 4 for outputting to the control means 3 time information indicating the current time.

The organic EL panel 2 includes a large number of cells. As illustrated in Fig. 1(b), each of the cells includes a glass substrate 5, an anode 6, an organic layer (second light emitter) 7 for emitting red light, an organic layer (third light emitter) 8 for emitting green light, an organic layer (first light emitter) 9 for emitting light having a dominant wavelength of approximately 464 nm, and a cathode 10.

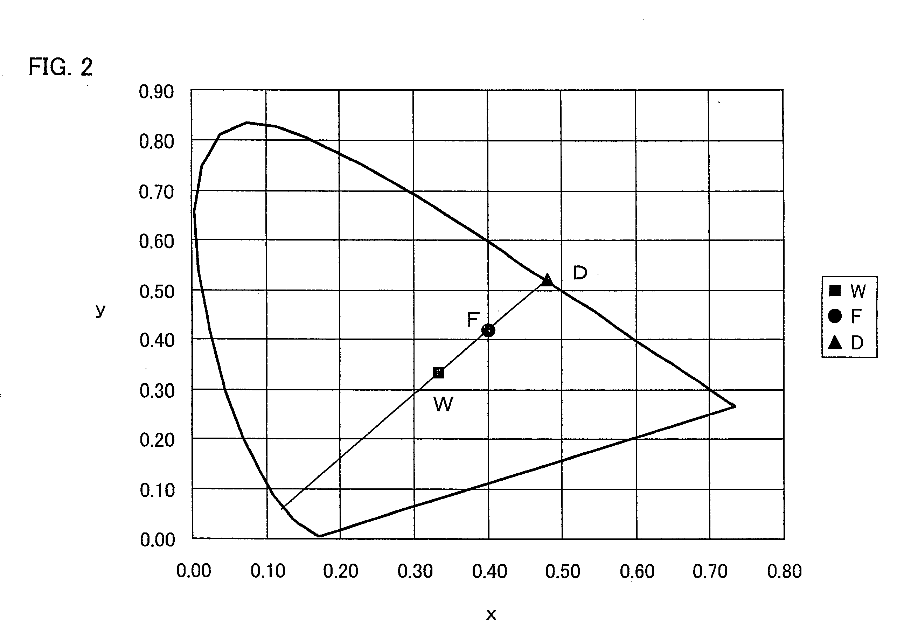

The meaning of the term "dominant wavelength" will be explained below. Suppose, as shown in Fig. 2, that a straight line connecting a white point W to a chromaticity point F intersects a spectrum locus at a point D, the white point W having an x-y coordinate of (x, y)=(1/3, 1/3) in an X-Y-Z color system, the color point F being a chromaticity point of light emitted from a light emitter. A dominant wavelength of the light emitter represented by the chromaticity point F means a wavelength of a monochromatic stimulus of light represented by the point D.

In Fig. 2, a heavy-line curve represents the spectrum locus, and points on the spectrum locus indicate the wavelengths of the monochromatic stimulus. Further, the white point W indicated herein serves as a reference to a color of a light source.

According to the foregoing arrangement, the display device 1 includes as a single pixel each of the cells of the organic EL panel 2 and performs color display by mixing respective colors of light beams emitted from the organic layers 7, 8, and 9.

Particularly, when the organic layer 9 is caused to emit light, the organic layer 9 emits light having a dominant wavelength of approximately 464 nm. This brings about a suppressive effect on melatonin secretion in a user of the display device 1, thereby awakening the user. The suppressive effect on melatonin secretion will be described in detail below.

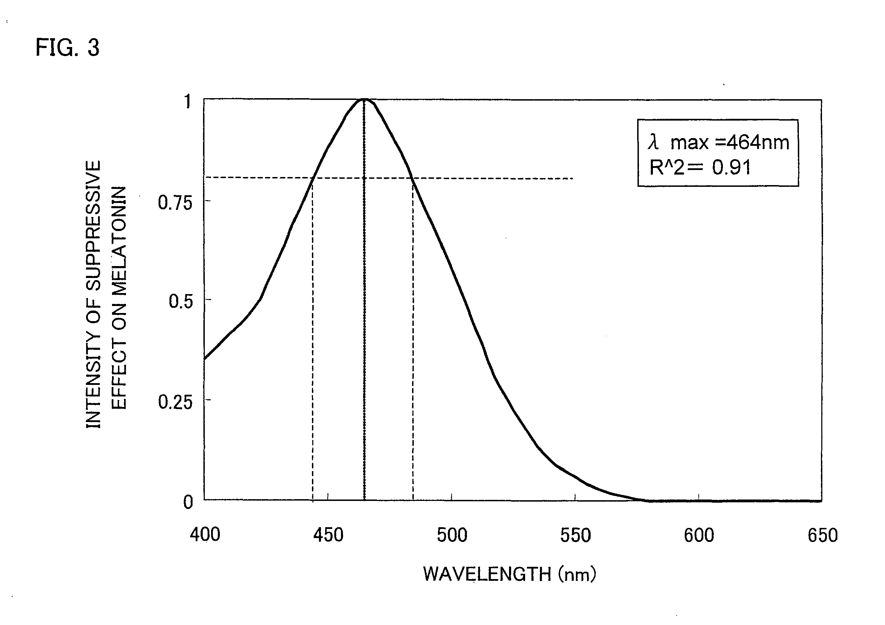

Fig. 3 is a graph showing a relationship between an emission wavelength and a suppressive effect on melatonin. In Fig. 3, the horizontal axis represents the emission wavelength, and the vertical axis represents an intensity of the suppressive effect on melatonin.

As shown in Fig. 3, the suppressive effect on melatonin is strong in a wavelength band corresponding to blue. More specifically, the suppressive effect on melatonin is strong in a wavelength range of 445 nm to 480 nm. Therefore, by using the organic layer 9 as a light emitter which has a dominant wavelength in this range, an effect of awakening the user can be obtained easily.

Particularly, as shown in Fig. 3, light having a wavelength of 464 nm has the strongest suppressive effect on melatonin. Therefore, by using the organic layer 9 as a light emitter whose light has a dominant wavelength of 464 nm, the effect of awakening the user can be obtained most effectively. Of course, also by using the organic layer 9 as a light emitter whose light is in a wavelength range of 445 nm to 480 nm, the effect of awakening the user can be obtained sufficiently.

The control means 3 illustrated in Fig. 1(a) brings about this awakening effect in the following manner. When the current time is determined to be in the daytime based on the time information obtained from the time output means 4 the control means 3 increases a current to be applied to the organic layer 9 illustrated in Fig. 1(b) so as to heighten an intensity of light emitted by the organic layer 9 based on an input video signal. This increases the proportion of an amount of light having a wavelength of 464 nm to the light emitted by the organic layers 7 to 9. In this way, it becomes possible to awaken the user.

Further, when the current time is determined to be in the nighttime based on time information obtained from the time output means 4 the control means 3 lowers an upper limit of a current to be applied to the organic layer 9 so as to lower an intensity of light emitted by the organic layer 9 based on an input video signal. This decreases the proportion of an amount of light having a wavelength of 464 nm to the light emitted by the organic layers 7 to 9. In this way, it is possible to reduce an effect of light emitted by the organic EL panel 2 on a biorhythm.

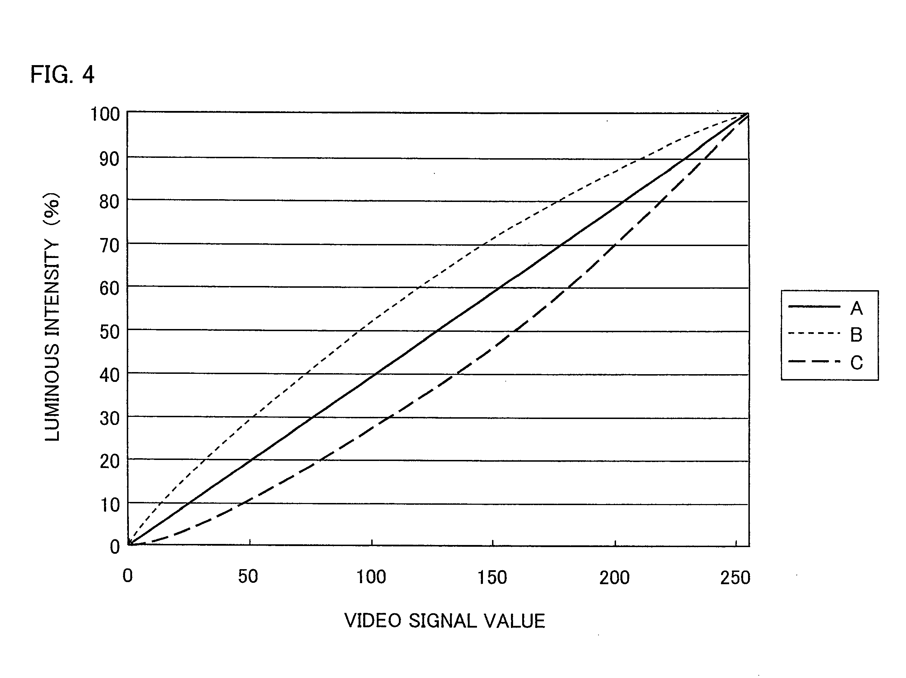

The process by which the control means 3 thus controls a luminous intensity of the organic layer 9 depending on whether the current time is in the daytime or nighttime will be described more in detail with reference to Fig. 4. Fig. 4 shows an emission characteristic of the organic layer 9 with respect to an input video signal. In Fig. 4, the horizontal axis represents a blue video signal value, and the vertical axis represents a luminous intensity.

In Fig. 4, the solid line A indicates a relationship between a video signal value and a luminous intensity when the organic layer 9 emits light without considering an effect on a biorhythm.

During the daytime when the user should be awakened, for example, the control means 3 is used to control the luminous intensity of the organic layer 9 so that, as indicated by the dotted line B in Fig. 4, a luminous intensity when the video signal value reaches its maximum and a luminous intensity when the video signal value reaches its minimum are the same as those indicated by the solid line A. Furthermore, in a range in which the video signal value is at its halftone level as indicated by the dotted line B in Fig. 4, the control means 3 is used to control the luminous intensity of the organic layer 9 so that the organic layer 9 emits light with a higher luminous intensity than that indicated by the solid line A.

By thus using the control means 3 to control the luminous intensity of the organic layer 9, the proportion of an amount of light having a wavelength of 464 nm to the light emitted by the organic layers 7 to 9 is increased. Therefore, it becomes possible to awaken the user.

Meanwhile, during the nighttime when the user should not be awakened, for example, the luminous intensity of the organic layer 9 in the range in which the video signal value is at its halftone level is made lower than that indicated by the solid line A, as indicated by the solid line C in Fig. 4. This decreases the proportion of an amount of light having a wavelength of 464 nm to the light emitted by the organic layers 7 to 9. Therefore, it is possible to reduce an effect of light emitted by the organic EL panel 2 on a biorhythm.

In Fig. 4, the luminous intensity of the organic layer 9 when the video signal value reaches its maximum is always constant regardless of whether or not the organic layer 9 emits light in consideration of an effect on a biorhythm. However, the luminous intensity does not need to be set in such a manner.

For example, a setting may be such that the luminous intensity of the organic layer 9 when the video signal value reaches its maximum is changed depending on whether or not the organic layer 9 emits light in consideration of an effect on a biorhythm, and the video signal value and the luminous intensity are proportionate to each other in the range in which the video signal value is at its halftone and low-gradation levels. Thus, various characteristics of the luminous intensity of the organic layer 9 can be adopted.

In Fig. 1, for the sake of ease of understanding, the magnitude relations between the components are exaggerated in terms of size in order to facilitate the understanding, and each of the components is not of actual size. Further, the organic EL panel 2 illustrated in Fig 1(b) has a cell structure in which the organic layers 7, 8, and 9 emit three different colors, respectively. However, the organic EL panel 2 is not to be limited to this arrangement.

For example, the organic EL panel 2 may be arranged such that an organic layer serving as a light emitter emits monochromatic light, such as blue light, whose color is converted by using a fluorescent material. Alternatively, the organic EL panel 2 may be a color-filter type arranged such that an organic layer serving as a light emitter emits white light whose color is converted through filters into three colors of red, green, and blue.

Further, the organic EL panel 2 may be arranged such that the organic layers 7, 8, and 9 are replaced by inorganic EL layers. Furthermore, the organic EL panel 2 may be replaced by an LED display which uses LEDs (light-emitting diodes) as a light emitter for emitting each color.

The present embodiment is arranged such that the luminous intensity of the organic layer 9 is changed by using the time information outputted by the time output means 4. However, the luminous intensity of the organic layer 9 may be controlled in accordance with elapsed time information and contents information. The elapsed time information indicates time elapsed from the beginning of use of the display device 1 to the present. The contents information indicates what type of program is displayed based on a video signal.

More specifically, when the luminous intensity of the organic layer 9 is adjusted based on the elapsed time information, the luminous intensity of the organic layer 9 is set higher than usual by using the control means 3, so that an awakening effect is brought about immediately after the beginning of use of the display device 1. As time elapses, the luminous intensity of the organic layer 9 is lowered by using the control means 3.

On this account, immediately after the user begins a task using the display device 1, e.g., a task of editing characters displayed by the display device 1, the luminous intensity of the organic layer 9 is set high, so that the user is awakened. Consequently, efficiency of the user's work can be improved immediately after the user begins the task using the display device 1, i.e., when the user tends to have trouble concentrating on the task.

Moreover, when some time has elapsed since the beginning of the task using the display device 1, the user concentrates on the task naturally, so that the efficiency of the user's work can be maintained without awakening the user. Therefore, when the luminous intensity of the organic layer 9 is lowered as time elapses, the display device 1 consumes less power.

Further, when a long time has elapsed since the beginning of the task, the user loses concentration and becomes sleepy, so that the efficiency of the user's work drops. Accordingly, when a given period of time has elapsed, the luminous intensity of the organic layer 9 may be heightened again. As described above, there are various patterns of using the elapsed time information.

Further, when the luminous intensity of the organic layer 9 is adjusted based on the contents information, the luminous intensity of the organic layer 9 may be heightened by using the control means 3 so as to awaken the user, under such conditions that it is determined, according to the contents information, that the image displayed based on the video signal is for example a movie. This makes it possible to cause a movie displayed by the display device 1 to be more impressive than when the luminous intensity of the organic layer 9 is not heightened. It is also possible that the user causes the display device 1 to memorize in advance by what type of image the user desires to obtain an awakening effect.

Further, the display device 1 may be provided with measuring means (not shown) for measuring an ambient luminance level, so that the luminous intensity of the organic layer 9 is controlled by the control means 3 in accordance with the luminance level measured by the measuring means. For example, the luminous intensity of the organic layer 9 is adjusted by using the control means 3 in the following manner. That is, when the display device 1 is in a bright environment, the luminous intensity of the organic layer 9 is heightened so as to awaken the user. Meanwhile, when the display device 1 is in a dark environment, the luminous intensity of the organic layer 9 is lowered.

### [Second Embodiment]

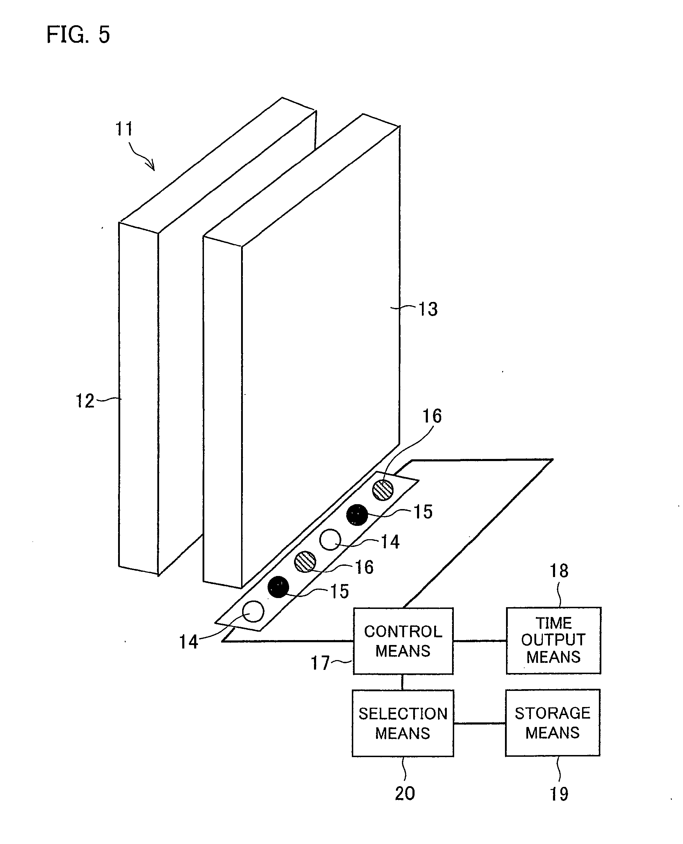

A display device according to another embodiment of the present invention will be described below with reference to Figs. 5 to 9. As illustrated in Fig. 5, a liquid crystal display device (display device) 11 of the present embodiment includes a liquid crystal panel (image display section) 12 for displaying information such as an image, an optical waveguide plate 13, LEDs (first light emitters) 14 each of which emits light having a dominant wavelength of approximately 464 nm, LEDs (second light emitters) 15 each of which emits red (R) light, and LEDs (third light emitters) 16 each of which emits green (G) light.

Each of the LEDs 14, 15, and 16 serves as a light emitter for the optical waveguide plate 13. The optical waveguide plate 13 transmits, to the liquid crystal panel 12, light emitted by the LEDs 14, 15, and 16.

Note that components having the same functions as those described in the foregoing embodiment are given the same reference numerals. In Fig. 5, for the sake of ease of understanding, the magnitude relations between the components are exaggerated in terms of size in order to facilitate the understanding, and each of the components is not of actual size.

Further, the liquid crystal display device 11 includes: control means 17 for controlling respective luminous intensities of the LEDs 14, 15, and 16 serving as light emitters; time output means 18 for outputting time information; storage means 19 for storing a plurality of control patterns of controlling the respective luminous intensities of the LEDs 14, 15, and 16; and selection means 20 for selecting a control pattern from among the plurality of control patterns. The term "control pattern" here means stored information which coordinates a luminous intensity of an LED with time.

The steps of controlling a luminous intensity of each of the light emitters will be described below. First, during use of the liquid crystal display device 11, at least one of the LED 14, the LED 15, and the LED 16 is turned on. Then, by adjusting the respective luminous intensities of the LEDs 14, 15, and 16, respective colors of light beams emitted from the LEDs 14, 15, and 16 can be mixed in the optical waveguide plate 13, so that white light is produced.

Moreover, when the white light serving as a reference is produced by mixing the respective light beams emitted from the LEDs 14, 15, and 16 are mixed in the optical waveguide plate 13, the luminous intensity of the LED 14 is set as a reference value. Furthermore, the control means 17 is used to heighten and lower the luminous intensity of the LED 14 with respect to the luminous intensity set at the reference value.

An example of the reference white light is a daylight color which is commonly used as a reference color in televisions, monitors, and other types of equipment and which has a correlated color temperature of approximately 6500 K (Kelvin). This daylight color is called the CIE (Commission Internationale l'Eclarirage) standard illuminant D65.

Further, examples of the reference white light are the standard illuminant C, which has a correlated color temperature of 6,774 K, the complementary standard illuminant D50, which has a correlated color temperature of approximately 5000 K; and other light beams having various correlated color temperatures.

In the present embodiment, the reference white light is set at a color temperature of 8500 K, and the respective luminous intensities of the LEDs 14, 15, and 16 are set so that white light having that color temperature is produced within the optical waveguide plate 13.

Furthermore, the selection means 20 refers to the storage means 19 so as to determine a control pattern of controlling a light emitter. The storage means 19 stores a plurality of control patterns of controlling the light emitter. One of the control patterns is a pattern in which the LED 14 emits intense light during the early morning hours when a user usually has just woken up and during the after-lunch hours when the user tends to become sleepy. Another one of the control patterns is a pattern in which the LED 14 emits intense light during the nighttime when there may be a night worker. The control means 17 controls a luminous intensity of the light emitter based on the selected control pattern and the time information outputted from the time output means 18.

In order to obtain an awakening effect on the user, the control means 17 is used to heighten the luminous intensity of the LED 14. However, a change in the luminous intensity of the LED 14 causes a white balance to change. However, as described below, the liquid crystal display device 11 is set to various white balances in accordance with applications. Therefore, even when the white balance changes, there is no problem in actually using the liquid crystal display device 11.

For example, in a display device for plate making and printing, the reference white light based on which an image is displayed is set at a correlated color temperature of 5000 K. Further, in a normal television or monitor, the reference white light is usually set at a high correlated color temperature of 9300 K. Furthermore, in a high-definition television, the reference white light is set a correlated temperature of 6500 K. In a recent television product, the reference white light is set at a high correlated color temperature of 12000 K.

Therefore, when the reference white light lies in a correlated color temperature range of 5000 K to 12000 K, a change in white balance does not cause a problem in using the liquid crystal display device 11, unless accurate color reproduction is required as in the case of evaluation of printed colors.

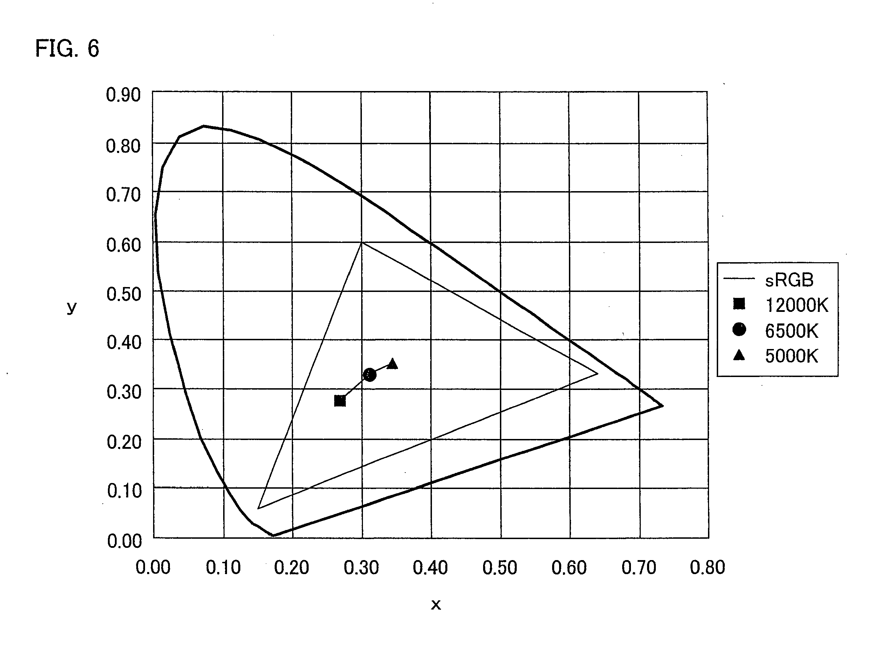

Fig. 6 shows how a chromaticity point of the reference white light based on which the liquid crystal display device 11 performs display moves on a chromaticity diagram when a color temperature of the reference white light is changed from 5000 K to 12000 K. In Fig. 6, the horizontal axis indicates a value of x in an X-Y-Z color system, the vertical axis indicates a value of y in the X-Y-Z color system.

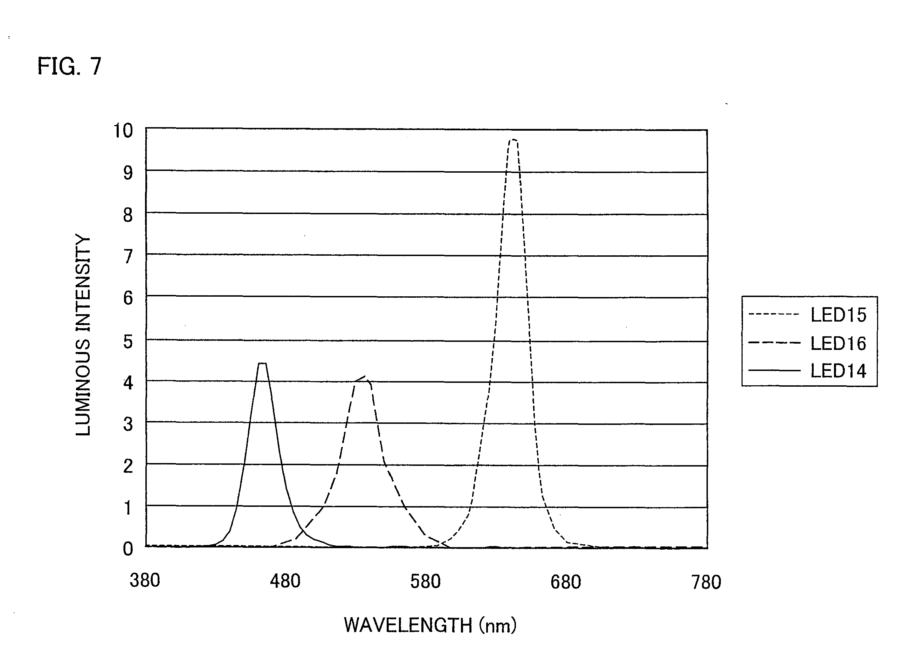

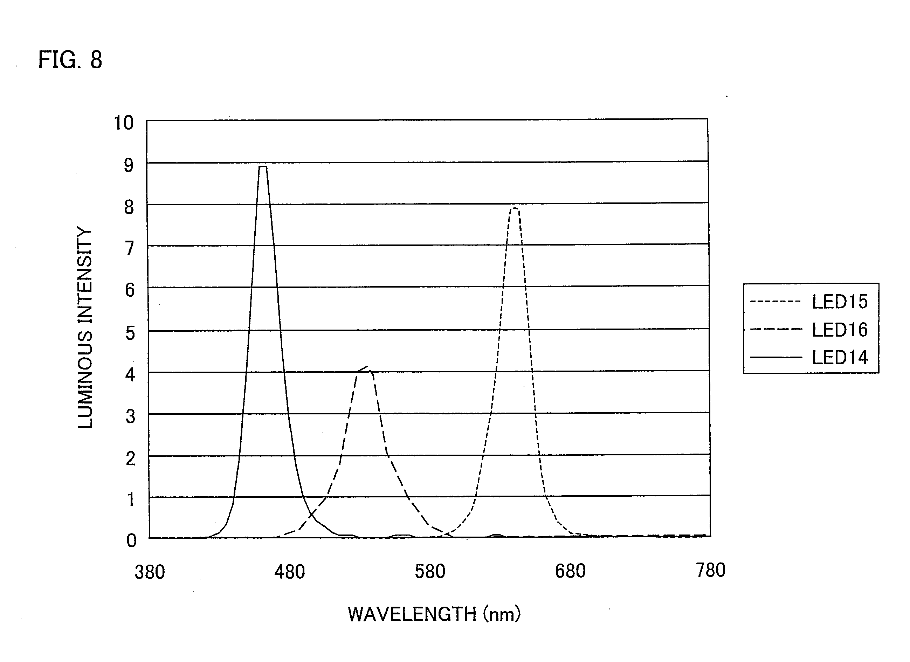

Further, Fig. 7 is a graph showing respective luminous intensities of the LEDs 14, 15, and 16 when the liquid crystal display device 11 is set at a correlated color temperature of 5000 K. Meanwhile, Fig. 8 is a graph showing respective luminous intensities of the LEDs 14, 15, and 16 when the liquid crystal display device 11 is set at a correlated color temperature of 12000 K.

In each of Figs. 7 and 8, the horizontal axis represents a wavelength, and the vertical axis represents the luminous intensity of each of the LEDs. Further, Figs. 7 and 8 have the same vertical scale.

A comparison of these two graphs shows that the luminous intensity of the LED 14, which emits blue light, differs between a case where the reference white light is set at a correlated color temperature of 5000 K and a case where the reference white light is set at a correlated color temperature of 12000 K. Specifically, the luminous intensity of the LED 14 when the reference white light is set at a correlated color temperature of 5000 K drops by approximately half when the reference white light is set at a correlated color temperature of 12000 K. Therefore, the luminous intensity of the LED 14 is greatly changed even when the correlated color temperature is changed within a range of actual use. This makes it possible to control suppression of melatonin so as to regulate a biorhythm.

There is a difference in the luminous intensity of the LED 15, which emits .red light, between Figs. 7 and 8. This applies to the case where the correlated color temperature is set at 5000 K and 12000 K on a blackbody radiation locus. When the white point does not need to be put on the blackbody radiation locus, the LED 15 does not need to be adjusted.

That is, the term "color temperature" expresses the color when a material called a blackbody is heated, as the temperature at which the black body is heated. The term "blackbody radiation locus" means a line which is drawn on a chromaticity diagram and which shows a change in chromaticity when the blackbody is heated. The term "correlated color temperature" means a color temperature which deviates from the blackbody radiation locus on the chromaticity diagram but which looks similar to the blackbody radiation locus. For adjusting only the correlated color temperature, it is only necessary to control the LED 14.

In order to obtain an effect of awakening the user, the luminous intensity of the LED 14 is heightened until the correlated color temperature reaches approximately 12000 K. In this case, a rapid change in the luminous intensity of the LED 14 may cause a rapid change in screen color balance so as to annoy the user of the liquid crystal display device 11. For this reason, when the LED 14 emits light with a high intensity, an output of the LED 14 is gradually increased in order to avoid a rapid change in screen color balance.

For example, if a PWM (Pulse Width Modulation) control is used to control an output of an LED in accordance with a period of time during which the LED emits light, a rapid change in screen color balance can be prevented by increasing the duty ratio in a certain period of time (e.g., five minutes). Further, the change in screen color balance can be prevented more effectively by heightening respective outputs of the LEDs 15 and 16 at the same time as the output of the LED 14.

Meanwhile, when the luminous intensity of the LED 14 is lowered in order to avoid an adverse effect on sleeping, the luminous intensity of the LED 14 is lowered so that the correlated color temperature of the reference color is approximately 5000 K. In the case of the PWM control, as described above, by lowering the duty ratio in a certain period of time (e.g., five minutes), the output of the LED 14 can be gradually lowered and a rapid change in screen color balance can be prevented. Further, by gradually lowering the respective outputs of the LEDs 15 and 16 at the same time as the output of the LED 14, the ratio of the respective luminous intensities of these three LEDs can be kept constant, so that a change in screen color balance can be prevented more effectively.

Described below is a relationship between an amount of light received by the user of the display device and a video signal value when the luminous intensity of the LED 14 is changed.

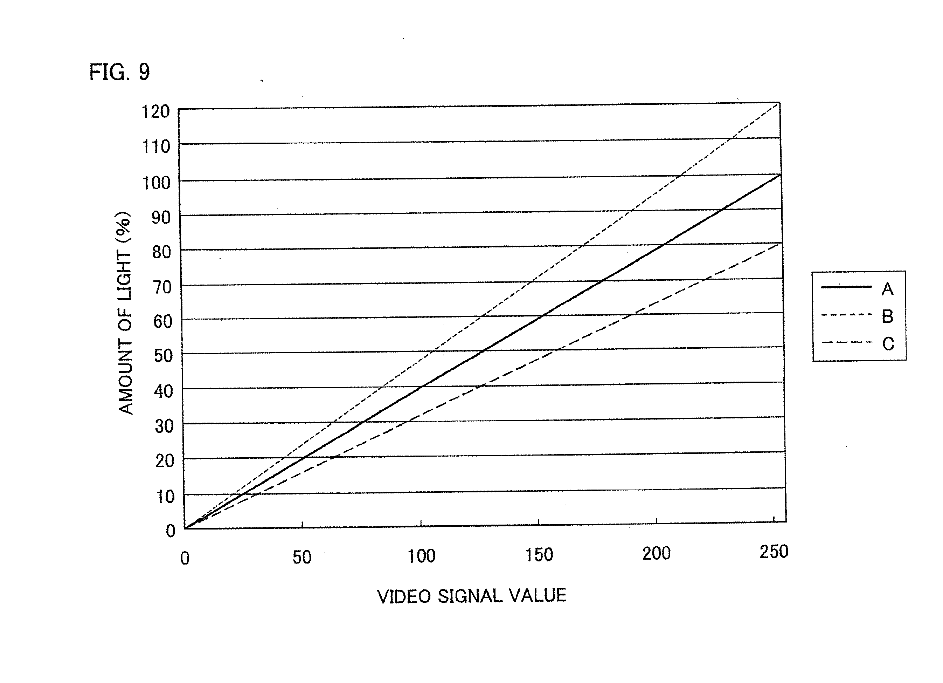

Fig. 9 is a graph showing a relationship between an amount of light received by the user and a video signal value. In Fig. 9, the solid line A represents a luminous intensity in normal times, the dotted line B represents a luminous intensity in an awakening period, and the dotted line C represents a luminous intensity in a sedative period for example before sleeping. The term "normal times" refers to a period of time during which the user may be awakened or may not be awakened. The term "awakening period" refers to a period of time during which the user should be awakened. The term "sedative period" refers to a period of time during which there should be no adverse effect on sleeping.

Suppose that the luminous intensity of the LED 14 is at 100% when the reference white light is set at a color temperature of 8500 K in the normal times. In this case, the luminous intensity of the LED 14 increases by 20% when the reference white light is set at a color temperature of 12000 K in the awakening period. Further, the luminous intensity of the LED 14 decreases by 20% when the reference white light is set at a color temperature of 5000 K in the sedative period.

The LEDs 14, 15, and 16 are used as backlights, so that their respective luminous intensities are kept constant in each of the normal times, the awakening period, and the sedative period. That is, in the case of the LED 14 for example, the luminous intensity of the LED 14 is always kept constant at 120% in the awakening period.

As shown in Fig. 9, in the awakening period for example, the amount of light reaching the user of the display device, as with the luminous intensity of the LED 14, increases by 20% as compared with the amount of light received in the normal times. Further, in the sedative period, the amount of light reaching the user of the display device, as with the luminous intensity of the LED 14, decreases by 20% as compared with the amount of light received in the normal times. Thus, it becomes possible to regulate a biorhythm by changing the amount of light reaching the user.

As for the number of the light emitters, the number of LEDs each having a low luminous intensity may be increased in accordance with a difference in maximum luminous intensities of LEDs having respective colors. The number of LEDs required may be increased in accordance with a white point serving as a reference. For example, when a bluish white point having a high color temperature serves as a reference, the number of blue LEDs is increased. The number of LEDs may vary depending on a color serving as a reference.

Two or more LEDs having the same color may be juxtaposed. All the LEDs are not necessarily arranged in a line as illustrated in Fig. 5. The LEDs may be arranged with one LED out of line with another LED. The LEDs may be categorized by color, and the categorized LEDs having the same color may be arranged in a line. Further, as illustrated in Fig. 5, the LEDs 14, the LEDs 15, and the LEDs 16 do not need to be arranged in this order but may be arranged in any given order.

Further, in the present embodiment, the reference white light is set at a correlated color temperature of 5000 K to 12000 K. However, these values only indicate normal values, and the correlated color temperature of the reference white light may be set at a higher color temperature. Therefore, when a more remarkable effect of awakening the user is desired regardless of the appearance of colors on the screen of the liquid crystal display device 11, the reference white light may be set at a color temperature lower than 5000 K or higher than 12000 K.

Further, the luminous intensity of each of the LEDs 14 is set at 100% in the normal times, 120% in the awakening period, and 80% in the sedative period, but not necessarily limited in that way. When a remarkable effect of regulating a biorhythm is desired, the gap between the upper and lower limits of the luminous intensity of the LED 14 may be widened. Further, the luminous intensity of the LED 14 may be appropriately changed in accordance with the luminous efficiency of the LED, the color temperature of the reference white light, and other parameters.

### [Third Embodiment]

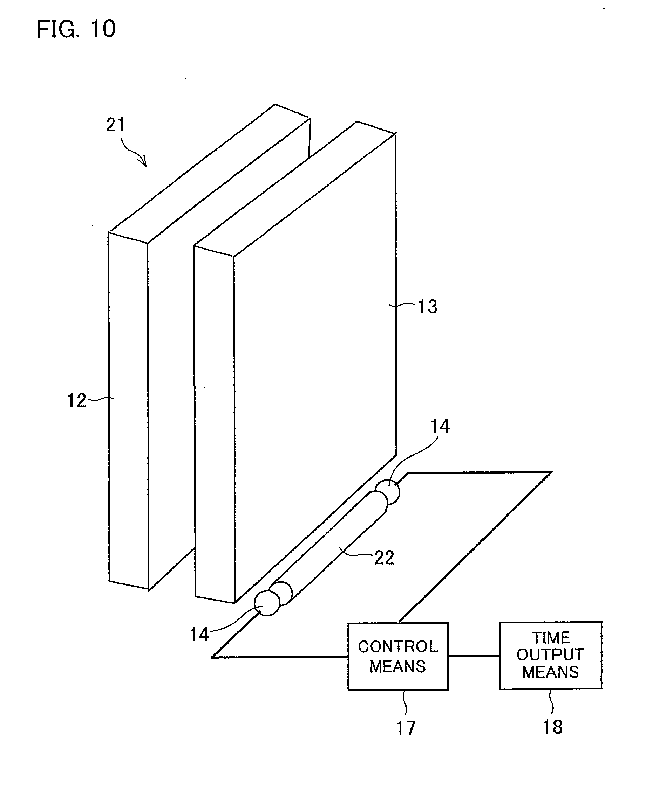

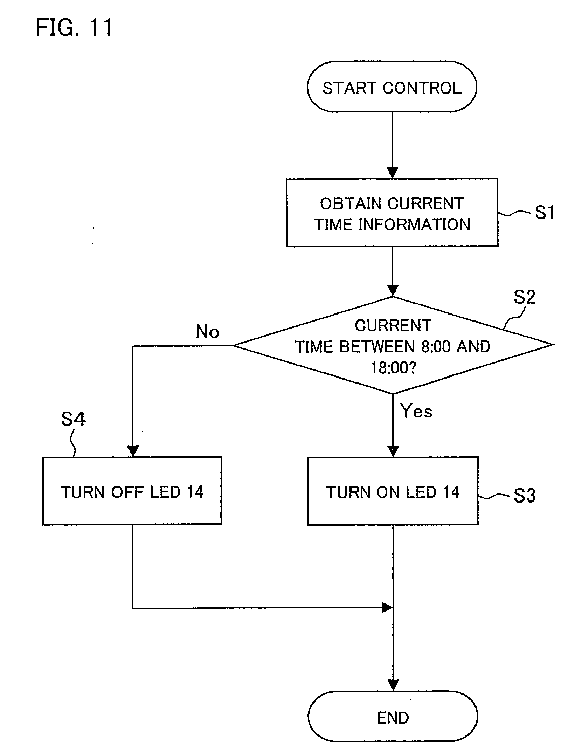

A display device according to a further embodiment of the present invention will be described below with reference to Figs. 10 and 11. A liquid crystal display device (display device) 21 of the present embodiment includes a liquid crystal panel 12 for displaying information such as an image, an optical waveguide plate 13 for irradiating a back surface of the liquid crystal panel 12, a cold cathode fluorescent lamp (white light emitter) 22, LEDs 14 each of which has a dominant wavelength of approximately 464 nm, control means 17 for controlling a luminous intensity of the LED 14, and time output means 18 for outputting current time information, each of the cold cathode fluorescent lamp 22 and the LEDs 14 serving as a light emitter for the optical waveguide plate 13.

A process will be described below by which the luminous intensity is controlled in the liquid crystal display device 21 of the foregoing arrangement. During use of the display device, the cold cathode fluorescent lamp 22 is always turned on. The cold cathode fluorescent lamp 22 emits white light, and the white light causes the optical waveguide plate 13 to look white.

Before starting to control the luminous intensity, the control means 17 obtains the current time information from the time output means 18. The control means 17, which controls the light emitter, controls the light emitter in the following manner, based on the time information.

That is, when the current time is in the daytime, the control means 17 turns on the LED 14. Further, when the current time is in the nighttime, the control means 17 turns off the LED 14 in order to prevent light of the LED 14 from affecting sleeping.

An LED is used as a light emitter having an emission wavelength of approximately 464 nm. This is because, as clearly shown in Fig. 7, an LED has a sharp spectrum, which makes it easy to limit its emission wavelength band. That is, by using an LED as a light emitter, a wavelength of light emitted from the light emitter can be limited to a wavelength of approximately 464 nm which greatly affects a biorhythm, so that it is possible to effectively regulate the biorhythm.

Fig. 11 is a flow chart explaining the foregoing process by which the luminous intensity is controlled. It is assumed here that the daytime is between eight and eighteen. As shown in Fig. 11, the control means 17 obtains the current time information from the time output means 18 (Step 1: hereinafter, "Step" will be referred to simply as "S").

Thereafter, the control means 17 determines whether or not the current time is a point of time between eight and eighteen (S2). The time between eight and eighteen is only an example of the time set as a daytime period of time and may be another period of time.

When it is determined in S2 that the current time is a point of time between eight and eighteen, the LED 14 is turned on so as to suppress melatonin secretion (S3). Meanwhile, when it is determined in S2 that the current time is out of the period of time between eight and eighteen, the LED 14 is turned off (S4) so as to reduce an effect on a biorhythm.

In Fig. 10, for the sake of ease of understanding, the magnitude relations between the components are exaggerated in terms of size in order to facilitate the understanding, and each of the components is not of actual size. Further, the LEDs 14, both of which are disposed at both ends, may be disposed at the center and may be increased in number. The cold cathode fluorescent lamp 22, which is disposed between the two LEDs 14, may not be provided between the two LEDs 14 and may be increased in number. Furthermore, the cold cathode fluorescent lamp 22 may be replaced by a white LED or an organic EL light emitter.

Furthermore, the LEDs 14 each of which has a dominant wavelength of 464 nm may be replaced by a light emitter which uses a fluorescent material to convert, into a wavelength of 464 nm, a wavelength of light from a light source having a different dominant wavelength. Further, a backlight LCD is taken for example in the present embodiment, but a frontlight may be used or a direct type backlight which has no optical waveguide plate and which is used in a large-sized LCD may be used.

### [Fourth Embodiment]

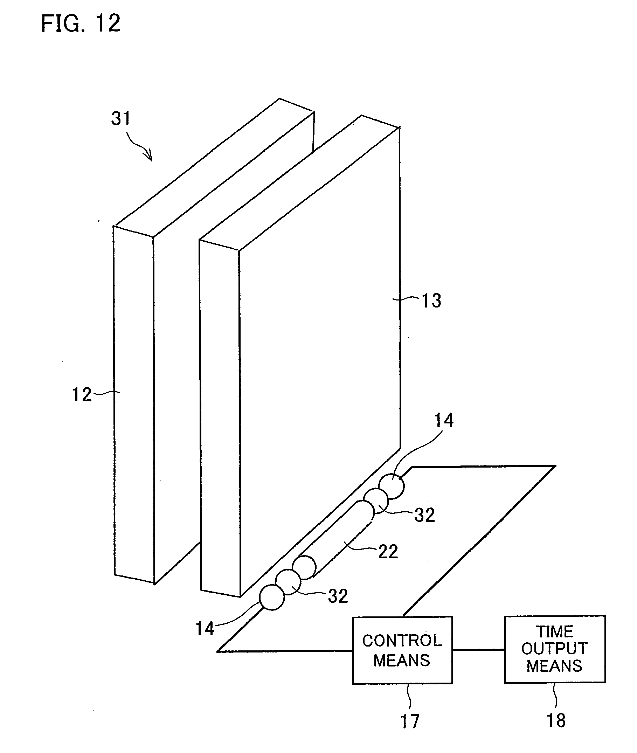

A display device according to a further embodiment of the present invention will be described below with reference to Figs. 12 to 14. As illustrated in Fig. 12, a liquid crystal display device (display device) 31 of the present embodiment includes a liquid crystal panel 12, an optical waveguide plate 13 for irradiating the liquid crystal panel 12, a cold cathode fluorescent lamp 22 serving as a light emitter, LEDs 14 each of which serves as a light emitter having a dominant wavelength of approximately 464 nm, LEDs (complementary light emitters) 32 each of which serves as a light emitter having a dominant wavelength complementary to the corresponding LED 14, control means 17 for controlling a luminous intensity of each of the light emitters, and time output means 18 for outputting current time information.

Note that components having the same functions as those described in the foregoing embodiments are given the same reference numerals. Further, in Fig. 12, for the sake of ease of understanding, the magnitude relations between the components are exaggerated in terms of size in order to facilitate the understanding and each of the components is not of actual size.

According to the present embodiment, as with the Third Embodiment, during use of the liquid crystal display device 31, the cold cathode fluorescent lamp 22 is turned on. Moreover, the control means 17 obtains the current time information from the time output means 18 and controls the luminous intensity of the light emitter based on the current time information.

For example, when the control means 17 receives a control pattern assuming that a user of the liquid crystal display device 31 is assigned to night duty, the control means 17 turns on each of the LEDs 14 before and during duty hours so as to suppress melatonin secretion, thereby awakening the user. For example, when it is assumed that the user is on duty at twenty or later, the LED 14 may be turned on few hours earlier, e.g., at eighteen so as to awaken the user.

At this time, the LED 14 is turned on so as to change a color balance on the optical waveguide plate 13, thereby changing a screen color balance. Accordingly, each of the LEDs 32 is turned on. The LED 32 is disposed next to the LED 14 and emits light serving as a complementary color to a color of light having a dominant wavelength of approximately 464 nm. Because the LED 14 and the LED 32 have colors complementary to each other, white light is produced by mixing light beams emitted by these two LEDs. This makes it possible to prevent great disruption of a white balance while increasing light having a wavelength component of 464 nm, thereby making it possible to reduce a change in screen color balance.

Specifically, a luminous intensity of the LED 32 is controlled so that a correlated color temperature of the white light, which is obtained by mixing the light emitted by the LED 14 and the light emitted by the LED 32, is as close as possible to a correlated color temperature of a color of light emitted by the cold cathode fluorescent lamp 22.

For example, when it is assumed that the color temperature of the light emitted by the cold cathode fluorescent lamp 22 is 9000 K, the light emitted from the LED 32 needs to have a dominant wavelength of 568 nm in order that the light obtained by mixing the color of the light emitted from the LED 14 and the color of the light emitted from the LED 32 reaches a color temperature of 9000 K.

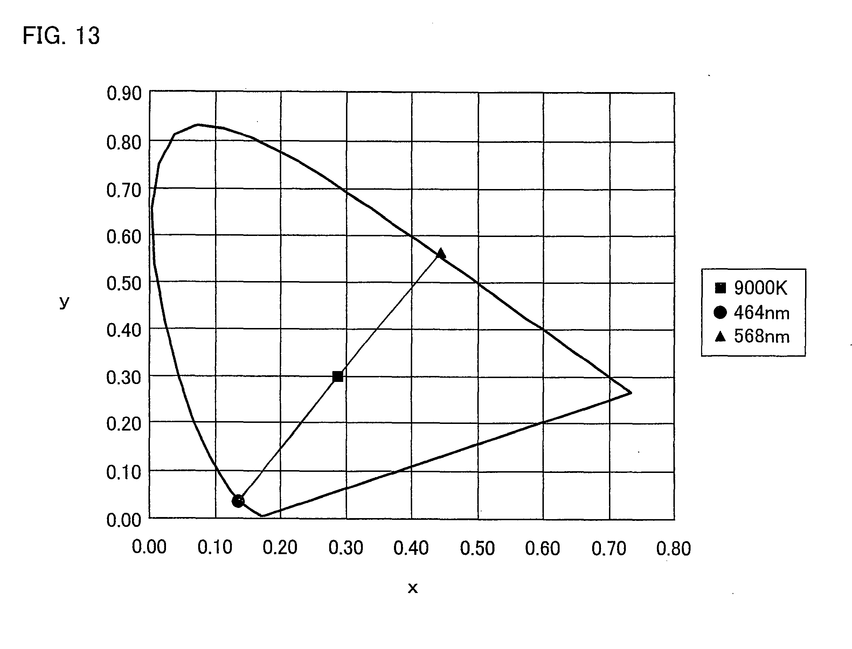

Fig. 13 shows a relationship between a dominant wavelength of the LED 14, a white point of the cold cathode fluorescent lamp 22, and a dominant wavelength of the LED 32, whose color is complementary to the color of the LED 14. The horizontal axis represents x in a X-Y-Z color system, and the vertical axis represents y in the X-Y-Z color system.

In Fig. 13, the curve indicated by the heavy line represents a spectrum locus, and each dot on the curve corresponds to a wavelength. The filled circle (indicated by • in the figure) indicates the dominant wavelength of 464 nm of the LED 14. The filled square (indicated by ■ in the figure) indicates the color temperature of the cold cathode fluorescent lamp 22. The filled triangle (indicated by ▲ in the figure) indicates a point where the straight line connecting the filled circle to the filled square intersects the spectrum locus. The point indicates the dominant wavelength of the LED 32 when the color temperature obtained by mixing the light emitted from the LED 32 with the light having a wavelength of 464 nm matches the color temperature of 9000 K of the cold cathode fluorescent lamp.

Thus, as long as the dominant wavelength of the LED 32 is set at 568 nm, adjusting the respective luminous intensities of the LEDs 14 and 32 makes it possible to cause the color of light obtained by mixing the light beams emitted from these two LEDs to have the same color temperature of 9000 K as the cold cathode fluorescent lamp 22.

Further, the control means 17 has various control patterns. In one control pattern, the LED 14 is turned off so as to regulate a biorhythm before the user has a catnap or when the user finishes working at daybreak. In another control pattern, if the user goes home by car, the LED 14 is kept on until the user finishes working. In these cases, when the LED 14 is turned on, the LED 32 is also turned on. Also, when the LED 14 is turned off, the LED 32 is also turned off.

For turning on the LEDs 14 and 32, respective outputs of the LEDs 14 and 32 are gradually changed in the same manner as in the Third Embodiment. This is because when these LEDs are turned on suddenly, a screen luminance rapidly changes.

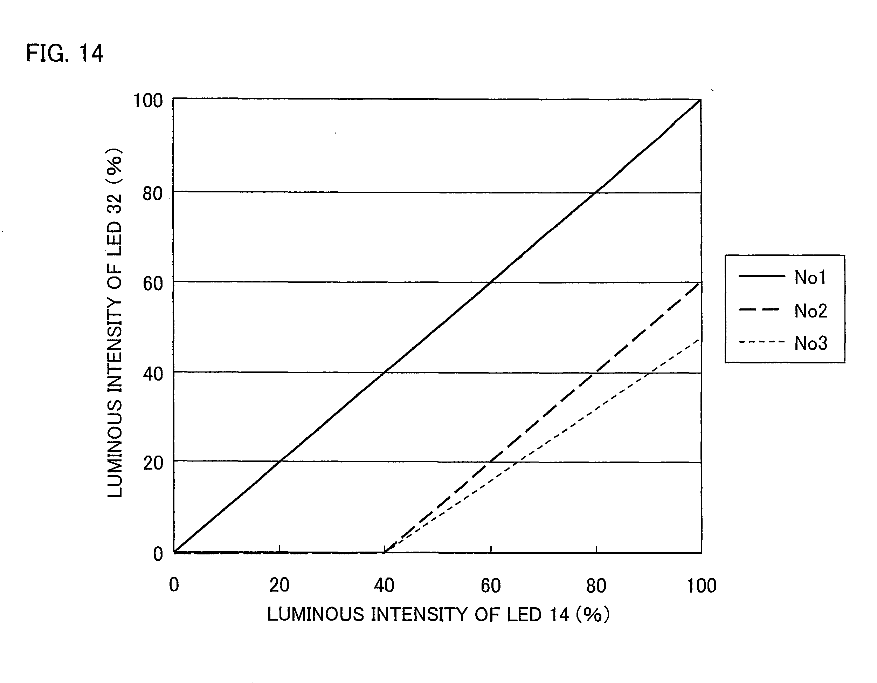

Fig. 14 shows changes in respective luminous intensities of the LED 14 and the LED 32 when these two LEDs are turned on. The horizontal axis represents the luminous intensity of the LED 14, and the vertical axis represents the luminous intensity of the LED 32.

The control means 17 changes the respective luminous intensities of the LEDs 14 and 32 so that the intensity of the LED 32 is changed in accordance with the intensity of the LED 14 as indicated by the solid line No. 1. Further, the control means 17 changes the respective luminous intensities of the LEDs 14 and 32 so that the LED 32 is turned off when the LED 14 is turned off. The respective luminous intensities of the LED 14 and the LED 32 are set so that the white balance becomes appropriate when the LED 14 and the LED 32 emit light at the same percentage of luminous intensity with respect to their respective maximum luminous intensities.

However, when the respective luminous intensities of the LED 14 and the LED 32 are heightened, the luminance of the entire screen is increased, so that the screen may look glaring. This can be prevented by lowering the luminous intensity of the cold cathode fluorescent lamp 22 when increasing the respective luminous intensities of the LED 14 and the LED 32. This makes it possible to suppress an increase in luminance, thereby reducing power consumption.

In Fig. 14, the luminous intensities of the two LEDs 14 and 32 are proportionate to each other. However, due to an emission characteristic and other differences in LED characteristic, the luminous intensities may not be proportionate to each other but may be expressed, for example, as a quadratic function curve.

Further, although the color of the light emitted by the cold cathode fluorescent lamp 22 is set at 9000 K in the present embodiment, the color temperature of the color of the light emitted by the cold cathode fluorescent lamp 22 may be set at various correlated color temperatures other than 9000 K. Therefore, the dominant wavelength of the LED 32 may be set at various wavelengths depending on the color temperature of the cold cathode fluorescent lamp 22 and the wavelength of the LED 14.

Further, ideally speaking, it is preferable that the dominant wavelength of the LED 32 be set at a wavelength serving as a complementary color to the color of the light emitted by the LED 14. However, because a wavelength slightly out of phase with the wavelength serving as the complementary color can emit white light approximate to that of the cold cathode fluorescent lamp 22, the LED 32 may be replaced by an LED which emits light having such a wavelength.

Further, the LED 32 emits monochromatic light having a spectrum which includes a single sharp peak. However, the LED 32 may be replaced by a light source which emits non-monochromatic light having an emission spectrum which includes a plurality of peaks and which has a dominant wavelength serving as a complementary wavelength.

### [Fifth Embodiment]

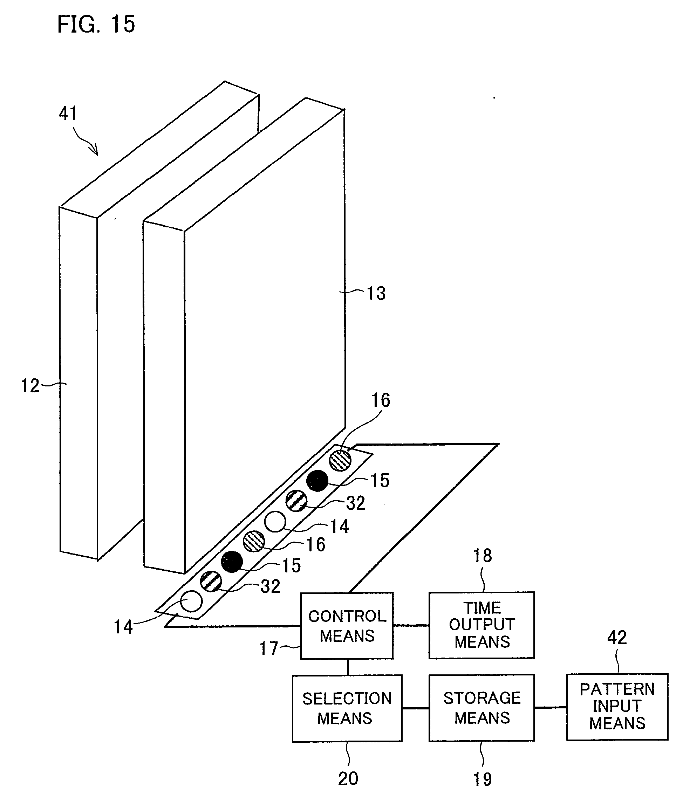

A display device according to a further embodiment of the present invention will be described below with reference to Fig. 15. As illustrated in Fig. 15, a liquid crystal display device (display device) 41 of the present embodiment includes a liquid crystal panel 12, an optical waveguide plate 13, LEDs 14, red (R) LEDs 15, green (G) LEDs 16, and LEDs 32. Each of the LEDs 14 emits light having a dominant wavelength of approximately 464 nm, and each of the LEDs 32 emits light serving as a complementary color to the light having a wavelength of approximately 464 nm. Further, the LEDs 14, 15, 16, 32 serve as light emitters for the optical waveguide plate 13.

Furthermore, the liquid crystal display device 41 includes control means 17 for controlling the light emitters, time output means 18 for outputting time information, storage means 19 for storing a plurality of control patterns of controlling the light emitters, pattern input means 42 for enabling a user of the display device to arbitrarily input a control pattern, and selection means 20 for selecting a control pattern to be used from among the plurality of control patterns.

Further, components having the same functions as those described in the foregoing embodiment are given the same reference numerals. Further, in Fig. 15, for the sake of ease of understanding, the magnitude relations between the components are exaggerated in terms of size in order to facilitate the understanding, and each of the components is not of actual size.

The steps of controlling respective luminous intensities of the light emitters will be described below. First, during use of the liquid crystal display device 41, the LEDs 14, the red LEDs 15, and the green LEDs 16 are turned on in order to produce white light. The white light at this time is set to the CIE standard illuminant D65. Furthermore, the selection means 20 refers to the storage means 19 so as to determine a control pattern of controlling the light emitters.

The storage means 19 stores the plurality of control patterns of controlling the light emitters as described above but has difficulty in storing all of the control patterns in advance. However, the liquid crystal display device 41 is provided with the pattern input means 42, which causes the storage means 19 to store, as user instruction information, the control pattern inputted by the user. This makes it possible for the user of the liquid crystal display device 41 to freely store control patterns in the storage means 19 in accordance with the user's lifestyle.

Note that the term "user instruction information" means information set by the user and regarding how a luminous intensity of the LED 14 is controlled in accordance with time. Therefore, the user instruction information also includes the control patterns of controlling the light emitters.

The user uses the selection means 20 to select one control pattern from among the plurality of control patterns which include the control pattern inputted by the user and which are stored in the storage means 19. The control means 17 controls the light emitters based on the selected control pattern and the time information outputted from the time output means 18.

Further, when the luminous intensity of the LED 14 is gradually heightened in order to obtain an effect of awakening the user, a screen color balance changes. In order to prevent such a change in color balance, the LED 32, which serves as a complementary color to the LED 14, is turned on in accordance with the luminous intensity of the LED 14, thereby making it possible to keep a white balance.

In Fig. 14, the dotted line No. 2 indicates a relationship between the respective luminous intensities of the LED 14 and the LED 32. The luminous intensity of the LED 14 and the luminous intensity of the LED 32 are set to be proportionate to each other, and the reference white light is set to D65.

At 40% of the luminous intensity of the LED 14, there is an appropriate white balance between the LED 14, the LED 15, and the LED 16. That is, when it is assumed that the reference white light D65 is emitted, the luminous intensity of LED 32 is kept at 0% until the intensity of the LED 14 reaches a predetermined intensity, e.g., 40%.

Meanwhile, when the luminous intensity of the LED 14 exceeds the predetermined intensity, the luminous intensity of the LED 32, whose color is complementary to the color of the LED 14, is caused to increase as the luminous intensity of the LED 14 increases. This makes it possible to keep the white balance properly, thereby reducing a change in screen color balance.

Further, also by adjusting respective outputs of the remaining two LEDs 15 and 16 in accordance with the luminous intensity of the LED 14, the white balance can be adjusted so that a change in color balance is restrained. These methods for restraining a change in color balance can be carried out alone or in combination. However, the method carried out by using the LED 32 serving as a complementary color allows for the easiest control with high accuracy.

Further, by disposing the LED 14 and the LED 32 next to each other, colors of light beams from these LEDs are mixed efficiently. This leads to reduction of unevenness in colors, thereby keeping the white balance more properly.

As luminous efficiency of an LED and a white balance serving as a reference vary, the graph changes in terms of slope and other characteristics. In Fig. 14, the dotted line No. 3 indicates an example of the relationship when the luminous intensity of the LED 32 is changed in accordance with the luminous intensity of the LED 14. The dotted line No. 3 indicates the relationship between the respective luminous intensities of the LED 14 and the LED 32 in the case where an appropriate white balance is achieved if the LED 32 has higher luminous efficiency and a lower luminous intensity than the LED 14.

Examples of the relationship between the respective luminous intensities of the LED 14 and the LED 32 include not only the relationship shown in Fig. 14 but also various relationships. For example, the luminous intensity of the LED 32 is kept at 0% until the luminous intensity of the LED 14 reaches 50%.

According to the present embodiment, the LED 14 and the LED 32 serving as a complementary color thereto are disposed next to each other in order to efficiently mix light beams from these LEDs. However, the LED 14 and the LED 32 may be disposed apart from each other.

Further, the arrangement of the liquid crystal display device 41 as an example of the display device has been described in the present embodiment. However, a white balance of a light-emitting display provided with pixels each of which has four colors including a complementary color can be adjusted in the same manner as the liquid crystal display device 41 of the present embodiment.

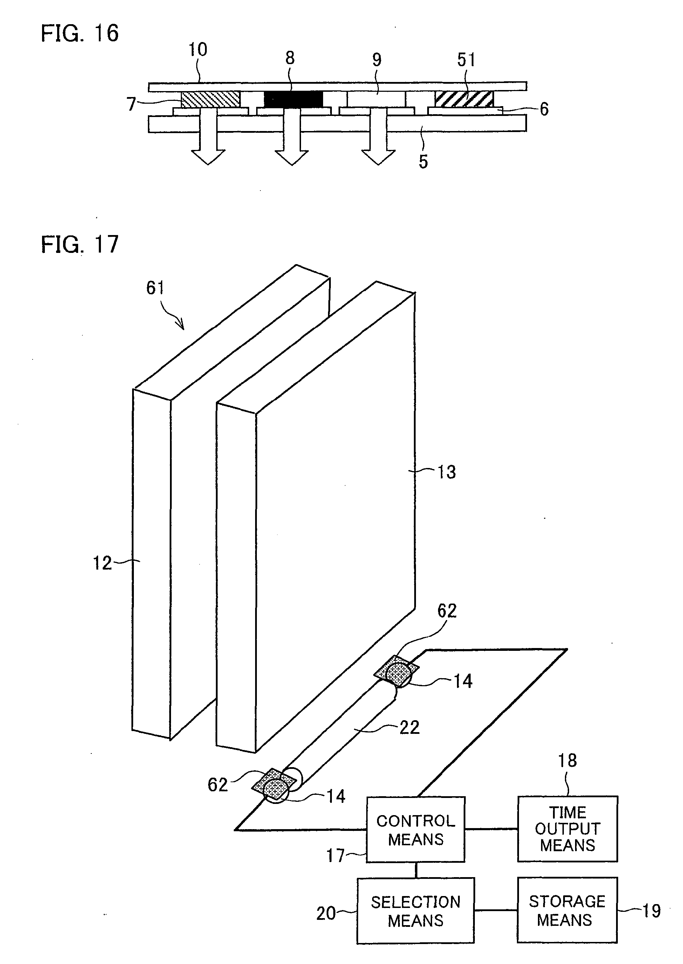

That is, as illustrated in Fig. 16, the light-emitting display includes: a glass substrate 5; an anode 6; an organic layer 7 for emitting red light; an organic layer 8 for emitting green light; an organic layer 9 for emitting light having a dominant wavelength of approximately 464 nm; and a cathode 10, wherein there is provided an organic layer (complementary light emitter) 51 for emitting light serving as a complementary color to the organic layer 9.

Further, Table 1 shows, for each of the various control patterns, emitting states of the LED 14 in each of the foregoing embodiments.

**[Table 1]**

| | **Pattern 1** | **Pattern 2** | **Pattern 3** | **· · ·** |
|---|---|---|---|---|
| **6:00-8:00** | **-** | **-** | **LOW** | |
| **8:00-10:00** | **ON** | **HIGH** | **-** | |
| **10:00-12:00** | **ON** | **HIGH** | **-** | |
| **12:00-14:00** | **ON** | **HIGH** | **-** | |
| **14:00-16:00** | **ON** | **HIGH** | **-** | |
| **16:00-18:00** | **ON** | **HIGH** | **-** | |
| **18:00-20:00** | **OFF** | **LOW** | **-** | |
| **20:00-22:00** | **OFF** | **LOW** | **-** | |
| **22:00-24:00** | **OFF** | **LOW** | **HIGH** | |
| **0:00-2:00** | **-** | **-** | **HIGH** | |
| **2:00-4:00** | **-** | **-** | **HIGH** | |
| **4:00-6:00** | **-** | **-** | **LOW** | |

Pattern 1 is a control pattern of controlling the light emitter described in the Third Embodiment and shows emitting states of the LED 14 with time. The liquid crystal display device 21 according to the Third Embodiment uses the cold cathode fluorescent lamp 22 as a light emitter (see Fig. 10), so that it is possible to produce white light without always keeping on the LED 14. Therefore, in Pattern 1, the LED 14 is turned on and off.

Further, Pattern 2 and Pattern 3 are control patterns of controlling the LED 14 when only three LEDs having their respective colors are used as light emitters. In these patterns, the LED 14 needs to be always kept on in order to produce white light. Therefore, in Pattern 2 and Pattern 3, the LED 14 is kept on with high and low intensities.

Further, Pattern 2 is suited to a normal life pattern, and Pattern 3 is suited to a life pattern for example of a person assigned to night duty. That is, in Pattern 2, the LED 14 is kept on with a high intensity during the daytime hours. Meanwhile, in Pattern 3, the LED 14 is kept on with a high intensity during the late night hours.

In addition to those patterns shown in Table 1, various control patterns can be set in accordance with situations in which the user of the display device lives. Further, in Table 1, the LED 14 is turned on and off and kept on with high and low intensities at two-hour intervals. However, time intervals at which the emission states of the LED 14 changes may be optionally determined.

### [Sixth Embodiment]

A display device according to a further embodiment of the present invention will be described below with reference to Fig. 17. As illustrated in Fig. 17, a liquid crystal display device (display device) 61 of the present embodiment includes a liquid crystal panel 12, an optical waveguide plate 13, LEDs 14 each of which has a dominant wavelength of approximately 464 nm, and a cold cathode fluorescent lamp 22. The LEDs 14 and the cold cathode fluorescent lamp 22 serve as light emitters for the optical waveguide plate 13. Further, the liquid crystal display device 61 of the present embodiment includes a fluorescent material 62, which is excited by light from the LEDs 14 so as to emit light serving as a complementary color to the LED 14.

Further, the liquid crystal display device 61 includes control means 17 for controlling respective luminous intensities of the light emitters (the LEDs 14, the cold cathode fluorescent lamp 22, and the fluorescent material 62), time output means 18 for outputting time information, storage means 19 for storing a plurality of control patterns of controlling the LEDs 14 serving as light emitters, and selection means 20 for selecting a control pattern to be used from among the plurality of control patterns.

Note that components having the same functions as those described in the foregoing embodiment are given the same reference numerals. Further, in Fig. 17, for the sake of ease of understanding, the magnitude relations between the components are exaggerated in terms of size in order to facilitate the understanding, and each of the components is not of actual size.

The steps of controlling the respective luminous intensities of the light emitters will be described below. First, during use of the liquid crystal display device 61, the cold cathode fluorescent lamp 22 is turned on in order to produce white light. The cold cathode fluorescent lamp 22 emits light having a correlated color temperature of 9000 K.

Furthermore, the selection means 20 refers to the storage means 19 so as to determine a control pattern of controlling the light emitters. The storage means 19 stores a plurality of control patterns. For example, in one of the plurality of control patterns, the LEDs 14 are caused to emit intense light in an early-morning period when a user has just woken up or in an after-lunch period when the user tends to be sleepy.

The user selects one control pattern from among the plurality of control patterns by using the selection means 20. The control means 17 controls the luminous intensity of each of the light emitters based on both the selected control pattern and the time information from the time output means 18. For example, when the user selects a pattern in which the LED 14 emits intense light with an eight-to-eighteen period set as a daytime period, the luminous intensity of each of the LEDs 14 is controlled according to the flow chart shown in Fig. 11.

Further, a color of light obtained by simply mixing light emitted by the cold cathode fluorescent lamp 22 with light emitted from the LED 14 is more bluish than a color of the light emitted by the cold cathode fluorescent lamp 22 alone. Therefore, in the liquid crystal display device 61 of the present embodiment, when the LED 14 is simply made to emit intense light in order to obtain an awakening effect on the user, the white balance is undesirably disrupted.

Accordingly, the fluorescent material 62 is disposed in a position which is between the LED 14 and the optical waveguide plate 13 and which is exposed to a part of the light from the LED 14. This causes the part of the light emitted from the LED 14 to be converted by the fluorescent material 62 into light serving as a complementary color to the LED 14. Further, another part of the light emitted from the LED 14 which does not pass through the fluorescent material 62 is not converted in wavelength and is therefore mixed with the light which has passed through the fluorescent material 62, thereby producing white light.

Thus, provision of the fluorescent material 62 makes it possible to, while heightening the intensity of the LED 14, adjust the white balance without using a light source of a complementary color. An example of such a fluorescent material is YAG (yttrium, aluminum, garnet), which is used for example in a white LED and emits yellow light when excited by blue light.

By thus arranging the liquid crystal display device 61, as compared with the liquid crystal display device 31 (see Fig. 12) using the LED 32 as a light emitter of a complementary color, light having the same luminance can be produced with a smaller number of light sources. Furthermore, when the fluorescent material 62 is used, the fluorescent material 62 is disposed in a position very near the LED 14, so that the liquid crystal panel 12 is less likely to display unevenness in colors and luminance.

Further, when a light emitter of a complementary color is used as with the liquid crystal display device 31 for example, a luminous intensity of the light emitter of the complementary color, as well as the luminous intensity of the LED 14, needs to be controlled. However, when the fluorescent material 62 is used as with the liquid crystal display device 61, there is no need to control a luminous intensity of the fluorescent material 62, so that the luminous intensity of the LED 14 can be controlled more simply than when a light emitter of a complementary color is used.

Although the fluorescent material 62 is disposed right above the LED 14 in the present embodiment, the fluorescent material 62 is not to be limited in terms of positions in which it is disposed. That is, the fluorescent material 62 can be disposed in various positions as long as it is exposed to the light from the LED 14. For example, the fluorescent material 62 can be disposed so as to cover the LED 14. Alternatively, the fluorescent material 62 can be disposed beside the LED 14.

Further, the LED 14 and the fluorescent material 62 may be replaced by a white LED obtained by combining with a fluorescent material an LED which emits light having a wavelength of 464 nm.

Further, although a cold cathode fluorescent lamp is used as a white light source in the present embodiment, a white LED or a white EL light emitter may be used. Further, a fluorescent material can be applied also to the liquid crystal display device 11 of the Second Embodiment using as light emitters the LEDs 14, 15, and 16 which emit different colors.

### [Seventh Embodiment]

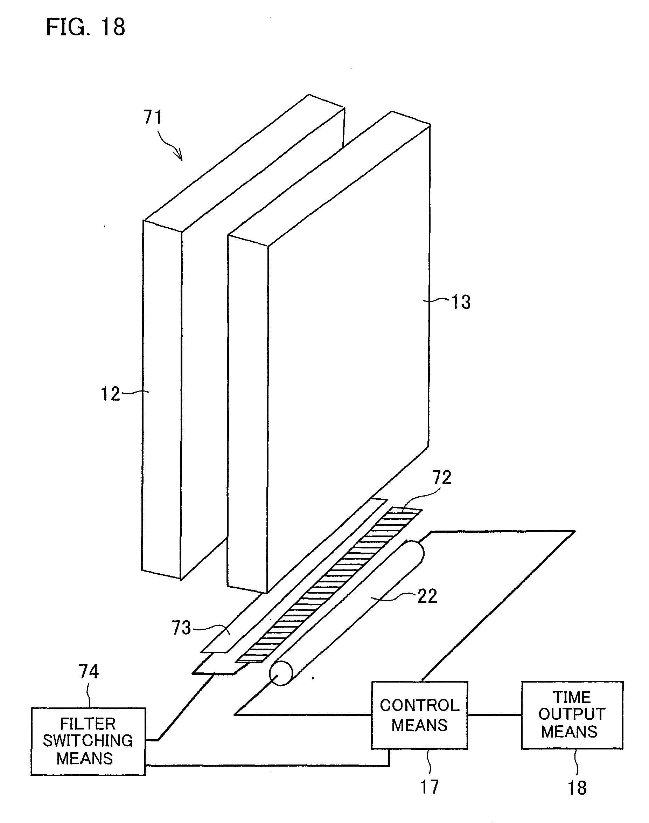

A display device according to a further embodiment of the present invention will be described below with reference to Fig. 18. As illustrated in Fig. 15, a liquid crystal display device (display device) 71 of the present embodiment includes a liquid crystal panel 12, an optical waveguide plate 13, and a cold cathode fluorescent lamp 22. The cold cathode fluorescent lamp 22 serves as a light emitter for the optical waveguide plate 13.

Further, the liquid crystal display device 71 includes a dielectric multilayer filter 72 which exhibits a low transmittance in a blue wavelength band, and a dielectric multilayer filter 73 which exhibits a high transmittance in the blue wavelength band. The dielectric multilayer filters 72 and 73 are disposed between the cold cathode fluorescent lamp 22 and that end face of the optical waveguide plate 13 which is irradiated with light of the cold cathode fluorescent lamp 22. The dielectric multilayer filters 72 and 73 control for each emission wavelength a transmittance of light incident to the optical waveguide plate 13 from the cold cathode fluorescent lamp 22.

Furthermore, the liquid crystal display device 71 includes filter switching means 74. The filter switching means 74 determines whether either one of the dielectric multilayer filters 72 and 73 is used or neither of them is used.

Further, the liquid crystal display device 71 includes control means 17 for controlling a luminous intensity of the cold cathode fluorescent lamp 22.

Note that components having the same functions as those described in the foregoing embodiments are given the same reference numerals. Further, in Fig. 18, for the sake of ease of understanding, the magnitude relations between the components are exaggerated in terms of size in order to facilitate the understanding, and each of the components is not of actual size.

Described below is a method for controlling an amount of light having such a wavelength in a range of 440 nm to 470 nm that affects a biorhythm.

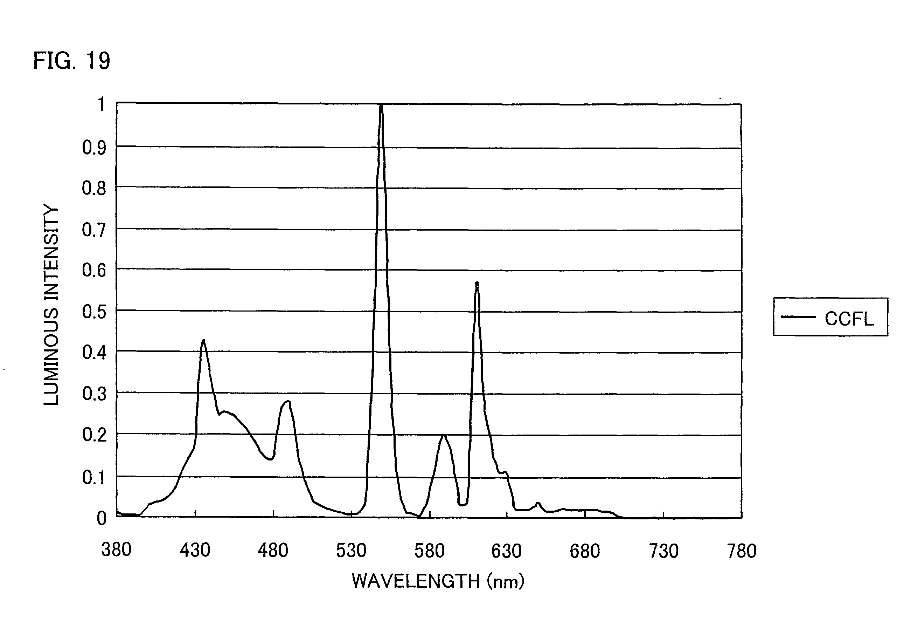

Fig. 19 shows an emission spectrum of the cold cathode fluorescent lamp 22. The horizontal axis represents the wavelength, and the vertical axis represents the luminous intensity. As described in Fig. 3, light in the blue wavelength band has a suppressive effect on melatonin. Fig. 19 shows that light emitted from the cold cathode fluorescent lamp 22 includes light in that wavelength band.

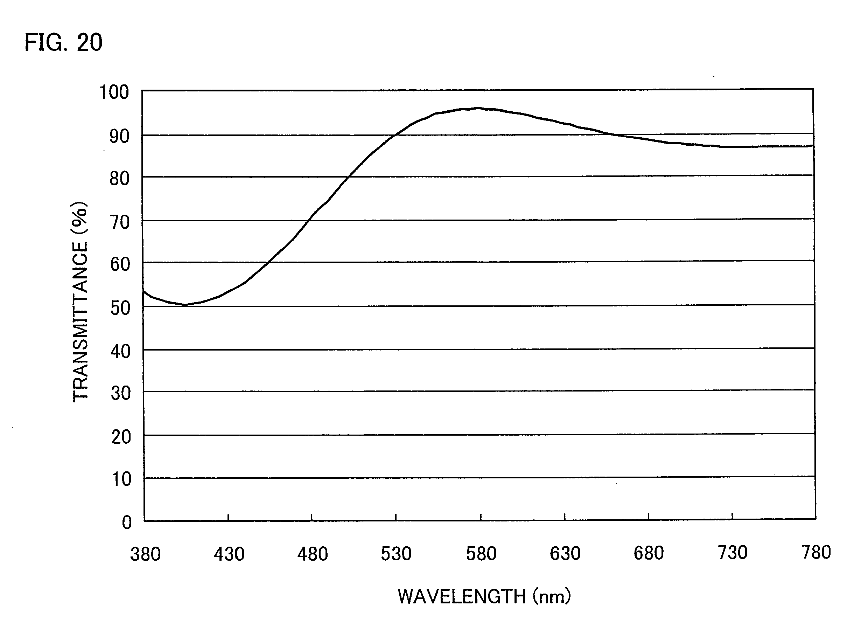

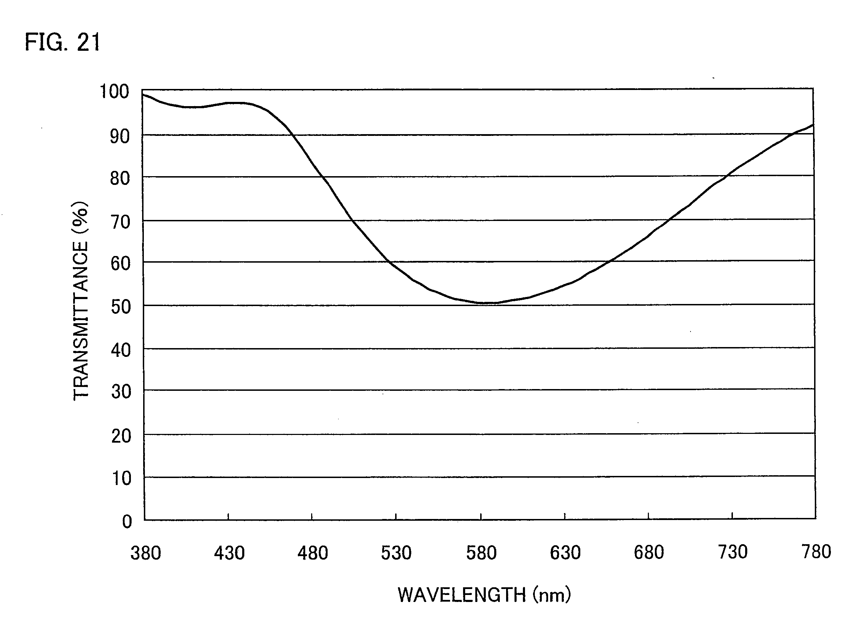

Further, Fig. 20 shows a transmittance characteristic of the dielectric multilayer filter 72, which exhibits a low transmittance in the blue wavelength band, and Fig. 21 shows a transmittance characteristic of the dielectric multilayer filter 73, which exhibits a high transmittance in the blue wavelength band. In each of Figs. 20 and 21, the horizontal axis represents the wavelength, and the vertical axis represents the transmittance.

The "dielectric multilayer filter" here includes two types of insulating films laminated on top of each other, the two types of insulating films having different refractive indices and being made for example of silicon oxide and titanium oxide. Dielectric multilayer filters having various transmission spectra can be achieved by changing materials of the insulating films or changing the number of layers laminated. There is a publicly known method for producing a dielectric multilayer filter.

As shown in Fig. 20, the dielectric multilayer filter 72 has a transmission spectrum which exhibits a low transmittance in the blue wavelength band and which exhibits a high transmittance in other wavelength bands. For this reason, in a period of time when the user should not be awakened, e.g., before sleeping, the switching means 74 is used to switch to the dielectric multilayer film 72. In this way, it is possible to reduce an amount of light which is in the blue wavelength band and has a suppressive effect on melatonin.

Further, as shown in Fig. 21, the dielectric multilayer filter 73 has a transmission spectrum which exhibits a high transmittance in the blue wavelength band and which exhibits a low transmittance in other wavelength bands. For this reason, in a period of time when the user needs to be awakened, e.g., in a daytime period, the switching means 74 is used to switch to the dielectric multilayer film 73.

However, using the dielectric multilayer film 73 alone causes an emission amount to decrease in all the wavelength bands, albeit at different rates between the blue wavelength band and the other wavelength bands. For this reason, when the dielectric multilayer filter 73 is used, it is preferable that the luminous intensity of the cold cathode fluorescent lamp 22 be heightened by using the control means 17. This makes it possible to increase an amount of light in the blue wavelength band so as to suppress melatonin secretion when the user needs to be awakened.

Furthermore, the user of the display device can use the filter switching means 74 so as to determine, as user instruction information, information regarding whether either one of the dielectric multilayer filters is used or neither of them is used.

For example, when the user does not want an adverse effect on sleeping before going to bed, the filter switching means 74 is used to set the user instruction information so that the dielectric multilayer filter 72, which exhibits a low transmittance in the blue wavelength band, is used. This makes it possible to prevent melatonin secretion from being suppressed.

Further, when there is a need for an effect of awakening the user, the filter switching means 74 is used to set the user instruction information so that the dielectric multilayer filter 73, which exhibits a high transmittance in the blue wavelength band, is used.

In this case, the filter switching means 74 outputs to the control means 17 a control signal to the effect that the luminous intensity of the cold cathode fluorescent lamp 22 is heightened. This causes an amount of light in the blue wavelength band to increase, so that an effect of awakening the user is obtained.

Further, when these filters are used, there is a change in amount of blue light, and this change may cause a change in color balance on a display screen of the liquid crystal display device 71. For this reason, when a color balance of a displayed image is important, it is preferable that the user use the filter switching means 74 so as to set the user instruction information so that neither of the filters is used. This makes it possible to prevent a color balance of an image displayed by the liquid crystal panel 12 from becoming bluish.

By thus setting the user instruction information by using the filter switching means 74, the user can set the liquid crystal display device 71 to a desired status.

In the present embodiment, dielectric multilayer filters are used because the dielectric multilayer filters make it possible to arbitrarily design a transmission spectrum in order to control an emission amount of light for each wavelength band. However, various methods may be used to control an emission amount of light for each wavelength band. For example, a guest-host liquid crystal may be used to control an emission amount.

The "guest-host liquid crystal" is a liquid crystal including a dichroic pigment mixed therein, the dichroic pigment being anisotropic in terms of light absorption between its major molecular axis and its minor molecular axis. Applying a voltage to the guest-host liquid crystal causes a change in alignment of the dichroic pigment. Because this change causes a change in absorption characteristic of light passing through the liquid crystal, an amount of transmission of light can be electrically controlled. A guest-host liquid crystal using a dichroic pigment which absorbs blue light makes it possible to control an amount of transmission of blue light.

Further, the cold cathode fluorescent lamp 22 may be replaced by an organic EL light emitter or an inorganic EL light emitter having an emission component in the blue wavelength band. Further, the number of the cold cathode fluorescent lamp 22 is not limited to one.

Further, in the present embodiment, the filter switching means 74 is provided for switching between the dielectric multilayer filter 72 and the dielectric multilayer filter 73. Similarly, there may be provided the organic layer 9, which emits blue light in the foregoing embodiments, and a button for changing the luminous intensity of the LED 14, so that the user can freely switch the luminous intensity.

The present invention is not to be limited by the foregoing embodiments and may be varied in many ways within the scope of the claims. Embodiments obtained by combining the technical means respectively disclosed in different embodiments are also included in the technical scope of the present invention.

As described above, a display device of the present invention is a display device for displaying an image based on light of a light emitter, wherein: the light emitter emits light having such a wavelength that affects a biorhythm, and an intensity of the light having the wavelength is controlled, so that the biorhythm is regulated and the image is displayed.

According to the foregoing arrangement, by controlling the intensity of the light having the wavelength which affects the biorhythm, the biorhythm can be regulated.

Particularly, because the display device is used in a position closer to a user than is a lighting device, the light from the light emitter directly reaches the user, thereby greatly affecting the user's biorhythm. Further, the display device for displaying the image is more frequently used than the lighting device.

Therefore, in the case of the display device, the luminous intensity of the light emitter has a more profound effect on the user's biorhythm than in the case of the lighting device. According to the present invention, the user's biorhythm can be effectively regulated.

Further, the display device of the foregoing arrangement preferably controls the intensity of the light having the wavelength, based time information. The time information embraces the time information indicating the current time, the elapsed time information indicating time elapsed from the beginning of use of the display device to the present, and other information.

[0172] According to the foregoing arrangement, the intensity of the light having the wavelength which affects the biorhythm is automatically controlled based on the time information, so that the user can regulate the biorhythm without consciously operating the display device.

Further, the display device of the foregoing arrangement preferably controls the intensity of the light having the wavelength, based on user instruction information set by the user.

According to the foregoing arrangement, the intensity of the light having the wavelength which affects the biorhythm is controlled based on the user instruction information set by the user. Therefore, the intensity of the light having the wavelength which affects the biorhythm can be controlled as the user likes, so that the user's biorhythm can be adapted to a desired rhythm.

Further, as the user instruction information, a control pattern may be set in which the intensity of the light having the wavelength which affects the biorhythm is associated with time, so that the intensity of the light having the wavelength which affects the biorhythm is controlled based on this user instruction information. Once the user of the display device of the present invention sets the control pattern, the light having the wavelength which affects the biorhythm is emitted with the user's desired intensity. Therefore, the user's biorhythm can be adapted to a desired rhythm.

Further, the display device of the foregoing arrangement preferably controls the intensity of the light having the wavelength, based on contents information indicating what type of program the displayed image is.

According to the foregoing arrangement, the intensity of the light having the wavelength which affects the biorhythm is controlled based on the contents information, so that the intensity of the light having the wavelength is controlled depending on what type of program the displayed image is. Therefore, the biorhythm can be regulated depending on what type of program the displayed image is. For example, when the displayed image is a movie, the intensity of the light having the wavelength is heightened so as to awaken the user, so that the movie becomes more impressive.

Further, the display device of the foregoing arrangement preferably includes a complementary light emitter for emitting light whose color is substantially complementary to a color of the light having the wavelength.

That is, when the intensity of the light having wavelength which affects the biorhythm is heightened, a color balance of a display screen on which the image is displayed may be disrupted. However, the complementary light emitter is provided in the foregoing arrangement. Therefore, by mixing light beams emitted from the light emitter and the complementary light emitter, white light is produced so that the color balance of the display screen does not change.

Further, the display device of the foregoing arrangement preferably controls a luminous intensity of the complementary light emitter in accordance with the luminous intensity of the light having the wavelength.

According to the foregoing arrangement, the luminous intensity of the complementary light emitter is controlled in accordance with the luminous intensity of the light having the wavelength. The complementary light emitter emits light whose color is complementary to the color of the light having the wavelength. Therefore, controlling the luminous intensity of the complementary light emitter in accordance with the luminous intensity of the light having the wavelength makes it possible to appropriately control the color balance of the white light, which is produced by mixing the light beams from the two light emitters.

Therefore, it is possible to more appropriately prevent the color balance of the display screen from being disrupted depending on the intensity of the light having the wavelength.

Further, a display device of the present invention is a display device including an image display section for displaying an image, the image display section including pixels each of which has a plurality of light emitters, wherein: the plurality of light emitters include a first light emitter for emitting light having such a wavelength that affects a biorhythm, and a characteristic of a luminous intensity of the first light emitter with respect to a video signal inputted into the image display section is switched.

According to the foregoing arrangement, the characteristic of the luminous intensity of the first light emitter is controlled in a light-emitting display device whose pixels serve as light emitters to emit light. This makes it possible to control the intensity of the light having the wavelength which affects the biorhythm, thereby regulating the biorhythm.

Particularly, because the display device is used in a position closer to a user than is a lighting device, the light from the first light emitter is directly reaches the user, thereby greatly affecting the user's biorhythm. Further, the display device for displaying the image is more frequently used than the lighting device.

Therefore, in the case of the display device, the luminous intensity of the first light emitter has a more profound effect on the user's biorhythm than in the case of the lighting device. According to the present invention, the user's biorhythm can be effectively regulated.

Further, a display device of the present invention is a display device including an image display section for displaying an image, the image display section including pixels each of which has a plurality of light emitters, wherein: the plurality of light emitters include a first light emitter for emitting light having a dominant wavelength in a range of 445 nm to 480 nm, and a characteristic of a luminous intensity of the first light emitter with respect to a video signal inputted into the image display section is switched.

According to the foregoing arrangement, a light emitter which emits light, which has a dominant wavelength in a range of 445 nm to 480 nm and has a high suppressive effect on melatonin, is used as the first light emitter. Therefore, light emitted from the first light emitter has a high suppressive effect on melatonin, thereby making it possible to more effectively regulate the biorhythm.

Further, the display device of the foregoing arrangement preferably switches the characteristic of the luminous intensity of the first light emitter with respect to the video signal, based on time information.

According to the foregoing arrangement, the characteristic of the luminous intensity of the first light emitter is automatically controlled based on the time information, so that the user can regulate the biorhythm without consciously operating the display device.

Further, the display device according to the foregoing arrangement preferably switches the characteristic of the luminous intensity of the first light emitter with respect to the video signal, based on user instruction information set by a user.

According to the foregoing arrangement, the characteristic of the luminous intensity of the light having the wavelength which affects the biorhythm is controlled based on the user instruction information set by the user. Therefore, the characteristic of the luminous intensity of the first light emitter can be controlled as the user likes, so that the user's biorhythm can be adopted to a desired rhythm.

Further, as the user instruction information, a control pattern may be set in which the characteristic of the luminous intensity of the first light emitter is associated with time, so that the characteristic of the luminous intensity of the first light emitter is controlled based on this user instruction information. Once the user of the display device of the present invention sets the control pattern, the first light emitter emits light with the user's desired intensity. Therefore, the user's biorhythm can be adopted to a desired rhythm.

Further, the display device according to the foregoing arrangement preferably switches the characteristic of the luminous intensity of the first light emitter with respect to the video signal, based on contents information indicating what type of program the displayed image is.

According to the foregoing arrangement, the characteristic of the luminous intensity of the first light emitter is controlled based on the contents information, so that the characteristic of the luminous intensity of the first light emitter is controlled depending on what type of program the displayed image is. Therefore, the biorhythm can be regulated depending on what type of program the displayed image is. For example, when the displayed image is a movie, the luminous intensity of the first light emitter is heightened so as to awaken the user, so that the movie becomes more impressive.

Further, the plurality of light emitters preferably include a second light emitter for emitting red light and a third light emitter for emitting green light.

That is, the light which is emitted by the first light emitter and which affects the biorhythm is substantially blue. Therefore, when the second light emitter and the third light emitter are provided as in the foregoing arrangement, color display can be performed in the light-emitting display device by using the substantially blue light emitted by the first emitter, the red light emitted by the second light emitter, and the green light emitted by the third light emitter.

Further, the plurality of light emitters preferably include a complementary light emitter for emitting light whose color is substantially complementary to a color of light emitted by the first light emitter.

That is, when the intensity of the light having wavelength which affects the biorhythm is heightened, a color balance of a display screen on which the image is displayed may be disrupted. However, the complementary light emitter is provided in the foregoing arrangement. Therefore, by mixing light beams emitted from the first light emitter and the complementary light emitter, white light is produced, so that the color balance of the display screen does not change.

Further, the display device of the foregoing arrangement preferably controls a luminous intensity of the complementary light emitter in accordance with the luminous intensity of the first light emitter.

According to the foregoing arrangement, the luminous intensity of the complementary light emitter is controlled in accordance with the luminous intensity of the first light emitter. The complementary light emitter emits light whose color is complementary to the color of the first light emitter. Therefore, controlling the luminous intensity of the complementary light emitter in accordance with the luminous intensity of the first light emitter makes it possible to appropriately control the color balance of the white light, which is produced by mixing the light beams from the two light emitters.

Therefore, it is possible to more appropriately prevent the color balance of the display screen from being disrupted depending on the luminous intensity of the first light emitter.

Further, the complementary light emitter is preferably disposed next to the first light emitter.

According to the foregoing arrangement, the first light emitter and the complementary light emitter are disposed next to each other, so that light beams emitted from the light emitters are efficiently mixed, thereby efficiently producing white light. Therefore, it is possible to more appropriately prevent the color balance of the display screen from being disrupted depending on the luminous intensity of the first light emitter.

Further, at least one of the plurality of light emitter may be a light-emitting diode.

According to the foregoing arrangement, a light-emitting LED display whose pixel includes an LED can be arranged so as to regulate a biorhythm.

Further, at least one of the plurality of light emitter may be an electroluminescent light emitter.

According to the foregoing arrangement, a light-emitting organic EL display whose pixel includes an electroluminescent light emitter can be arranged so as to regulate a biorhythm.

Further, a display device of the present invention is a display device irradiating an image display section, which is for displaying an image, with light from a light source so as to cause the image display section to display the image, the display device including: a plurality of light emitters which include a first light emitter for emitting light having such a wavelength that affects a biorhythm, a luminous intensity of the first light emitter with respect to an video signal inputted into the image display section being switched.

According to the foregoing arrangement, in the display device arranged such that the image display section is irradiated with the light from the light source, the luminous intensity of the first light emitter is controlled. This makes it possible to control the intensity of the light having the wavelength which affects the biorhythm, thereby regulating the biorhythm.

Particularly, because the display device is used in a position closer to a user than is a lighting device, the light from the first light emitter is sent directly to the user. Further, the display device for displaying the image is more frequently used than the lighting device.

Therefore, in the case of the display device, the luminous intensity of the first light emitter has a more profound effect on the user's biorhythm than in the case of the lighting device. According to the present invention, the user's biorhythm can be effectively regulated.

Further, a display device of the present invention is a display device irradiating an image display section, which is for displaying an image, with light from a light source so as to cause the image display section to display the image, the display device including: a plurality of light emitters which include a first light emitter for emitting light having a dominant wavelength in an range of 445 nm to 480 nm, a luminous intensity of the first light emitter with respect to an video signal inputted into the image display section being switched.

According to the foregoing arrangement, a light emitter which emits light, has a dominant wavelength in a range of 445 nm to 480 nm and has a high suppressive effect on melatonin, is used as the first light emitter. Therefore, light emitted from the first light emitter has a high suppressive effect on melatonin, thereby making it possible to more effectively regulate the biorhythm.

Furthermore, in the display device of the foregoing arrangement, it is preferable that the luminous intensity of the first light emitter be controlled based on time information.

According to the foregoing arrangement, the luminous intensity of the first light emitter is automatically controlled based on the time information, so that the user can regulate the biorhythm without consciously operating the display device.

Furthermore, in the display device of the foregoing arrangement, it is preferable that the luminous intensity of the first light emitter be controlled based on user instruction information set by the user.

According to the foregoing arrangement, the intensity of the light having the wavelength which affects the biorhythm is controlled based on the user instruction information set by the user. Therefore, the luminous intensity of the first light emitter can be controlled as the user likes, so that the user's biorhythm can be adapted to a desired rhythm.

Further, as the user instruction information, a control pattern may be set in which the luminous intensity of the first light emitter is associated with time, so that the luminous intensity of the first light emitter is controlled based on this user instruction information. Once the user of the display device of the present invention sets the control pattern, the first light emitter emits light with the user's desired intensity. Therefore, the user's biorhythm can be adapted to a desired rhythm.

Furthermore, in the display device of the foregoing arrangement, it is preferable that the characteristic of the luminous intensity of the first light emitter be controlled based on contents information indicating what type of program the image is.

According to the foregoing arrangement, the luminous intensity of the first light emitter is controlled based on the contents information, so that the luminous intensity of the first light emitter is controlled depending on what type of program the displayed image is. Therefore, the biorhythm can be regulated depending on what type of program the displayed image is. For example, when the displayed image is a movie, the luminous intensity of the first light emitter is heightened so as to awaken the user, so that the movie becomes more impressive.

Furthermore, the light source includes the light source preferably includes a second light emitter for emitting red light and a third light emitter for emitting green light.

That is, the light which is emitted by the first light emitter and which affects the biorhythm is substantially blue. Therefore, when the second light emitter and the third light emitter are provided as in the foregoing arrangement, white light can be produced by mixing the substantially blue light emitted by the first emitter, the red light emitted by the second light emitter, and the green light emitted by the third light emitter.

Moreover, the white light is emitted from a light source used in a commonly used display device. Therefore, a simple arrangement in which the first light emitter, the second light emitter, and the third light emitter are provided as described above causes these light emitters to function as a light source of a commonly used display device.

Furthermore, the light source may include a white light emitter for emitting white light.

That is, the white light is emitted from a light source used in a commonly used display device. Therefore, according to the foregoing arrangement, a simple arrangement in which a display device including a white light emitter used as a normal light source is provided with the first light emitter makes it possible to regulate a biorhythm.

Furthermore, the light source preferably includes a complementary light emitter for emitting light whose color is substantially complementary to a color of light emitted by the first light emitter.

That is, when the intensity of the light having wavelength which affects the biorhythm is heightened, a color balance of a display screen on which the image is displayed may be disrupted. However, the complementary light emitter is provided in the foregoing arrangement. Therefore, by mixing light beams emitted from the first light emitter and the complementary light emitter, white light is produced, so that the color balance of the display screen does not change.

Furthermore, in the display device of the foregoing arrangement, it is preferable that a luminous intensity of the complementary light emitter be controlled in accordance with the luminous intensity of the first light emitter.

According to the foregoing arrangement, the luminous intensity of the complementary light emitter is controlled in accordance with the luminous intensity of the first light emitter. The complementary light emitter emits light whose color is complementary to the color of the light emitter. Therefore, controlling the luminous intensity of the complementary light emitter in accordance with the luminous intensity of the first light emitter makes it possible to appropriately control the color balance of the white light, which is produced by mixing the light beams from the two light emitters.

Therefore, it is possible to more appropriately prevent the color balance of the display screen form being disrupted depending on the luminous intensity of the first light emitter.

Furthermore, the complementary light emitter is preferably disposed next to the first light emitter.

According to the foregoing arrangement, the first light emitter and the complementary light emitter are disposed next to each other, so that light beams emitted from the light emitters are efficiently mixed, thereby efficiently producing white light. Therefore, it is possible to more appropriately prevent the color balance of the display screen from being disrupted depending on the luminous intensity of the first light emitter.

Furthermore, the display device of the foregoing arrangement preferably includes a fluorescent material for emitting light whose color is substantially complementary to a color of light emitted by the first light emitter.

According to the foregoing arrangement, the fluorescent material emits the light whose color is substantially complementary to the color of the light emitted by the first light emitter, so that the fluorescent material brings about the same effect as the complementary light emitter. Further, there is no need to control a luminous intensity of the fluorescent material, so that the arrangement of the display device can be simplified.

Furthermore, at least one of the light emitters of the light source may be a light-emitting diode or an electroluminescent light emitter.

According to the foregoing arrangement, in a display device using as a light source a light-emitting diode or an electroluminescent light emitter, an image display section such as a liquid crystal panel is irradiated with light from the light source, thereby making it possible to regulate a biorhythm.

In addition, a display device of the present invention may include a first light emitter for emitting light having such a wavelength that affects a living organism and control means for controlling a luminous intensity of the first light emitter based on time information from time output means.

According to the foregoing arrangement, an intensity of light having such a wavelength that affects a living organism can be controlled based on time information obtained by time output means.

Therefore, a luminous intensity of light which affects a living organism can be adjusted in accordance with time, thereby making it possible to regulate a biorhythm.

Furthermore, the display device of the present invention is arranged such that the first light emitter emits light having a peak wavelength in a range of 445 nm to 480 nm.

It is known that melatonin secretion is suppressed by strongly emitting light having a peak wavelength of approximately 445 nm to 480 nm. According to the foregoing arrangement, the first light emitter emits light having a peak wavelength in the aforesaid range, so that melatonin secretion is suppressed. This makes it possible to more effectively regulate a biorhythm.

Furthermore, the display device of the present invention may include a second light emitter for emitting red light and a third light emitter for emitting green light.

According to the foregoing arrangement, sharp color display can be performed by using the first light emitter, the second light emitter, and the third light emitter.

Furthermore, the display device of the present invention may be arranged so as to include a fourth light emitter for emitting light whose color is complementary to a color of light emitted by the first light emitter.

According to the foregoing arrangement, the fourth light emitter emits light whose color is complementary to the color of light emitted from the first light emitter. Therefore, even when the first light emitter emits light with a high intensity, it is possible to reduce a change in screen color balance by causing the fourth light emitter to emit light with an accordingly high intensity.

Furthermore, the display device of the present invention may be arranged such that the fourth light emitter is disposed next to the first light emitter.

According to the foregoing arrangement, the first light emitter and the fourth light emitter are disposed next to each other, so that colors of light beams emitted from these two light emitters can be effectively mixed. This makes it possible to prevent unevenness in colors on the screen.

Furthermore, the display device of the present invention may be arranged such that the control means controls a luminous intensity of the fourth light emitter in accordance with the luminous intensity of the first light emitter.

According to the foregoing arrangement, the luminous intensity of the fourth light emitter is controlled in accordance with the luminous intensity of the first light emitter, so that a mixing ratio of respective colors of the first and fourth light emitters can be adjusted. This achieves a more ideal color balance on the screen.

Furthermore, the display device of the present invention may be arranged such that the first light emitter is included in lighting means for illuminating image display means for displaying an image.

According to the foregoing arrangement, a liquid crystal display or other displays commonly used as image display means is provided with the first light emitter serving as a light source of a backlight used as the lighting means. This makes it possible to easily provide a display device which regulates a biorhythm.

Furthermore, the display device of the present invention may be arranged such that at least the first light emitter, the second light emitter, and the third light emitter form a pixel of the image display means for displaying an image.

According to the foregoing arrangement, since the first light emitter, the second light emitter, and the third light emitter emit red light, green light, and blue light, respectively, the image display means can serve as a light-emitting display. Therefore, it is possible to provide a light-emitting display capable of regulating a biorhythm.

Further, the display device of the present invention may be arranged such that the control means changes the luminous intensity of the first light emitter in accordance with a control pattern selected by selection means from among a plurality of control patterns stored in storage means.

According to the foregoing arrangement, the storage means stores control patterns of various luminous intensities, the selection means selects a control pattern most appropriate to a user's daily rhythm, and the luminous intensity of the first light emitter can be changed in accordance with the selected control pattern.

Therefore, it is possible to more effectively regulate a biorhythm by setting a control pattern for each user of the display device.

Further, the display device of the present invention may be arranged such that the control means performs control so that that the luminous intensity of the first light emitter is heightened on rising of the user and in the daytime and is lowered in the nighttime.

According to the foregoing arrangement, the user can be more effectively awakened on rising and in the daytime and the luminous intensity is weakened before the user goes to bed. This reduces an effect on the user's biorhythm. Therefore, a biorhythm can be more effectively regulated.

Preferably, the first light emitter, the second light emitter, the third light emitter, and the fourth light emitter are LEDs. When each of light emitters is an LED, the light emitter can narrow down an emission wavelength, so that such a wavelength that affects a living organism can be accurately selected.

Further, the first light emitter, the second light emitter, the third light emitter, and the fourth light emitter may be electroluminescent light emitters. An electroluminescent light emitter makes it possible to provide an organic EL display capable of regulating a biorhythm.

The invention being thus described, it will be obvious that the same way may be varied in many ways. Such variations are not to be regarded as a departure from the spirit and scope of the invention, and all such modifications as would be obvious to one skilled in the art are intended to be included within the scope of the following claims.

### INDUSTRIAL APPLICABILITY

The present invention can be applied to various display devices such as monitors, television sets, displays for mobile devices, and large-screen displays.

## Claims

1. A display device for displaying an image by using light of a light emitter, wherein:
the light emitter emits light having such a wavelength that affects a biorhythm, and
an intensity of the light having the wavelength is controlled, so that the biorhythm is regulated and the image is displayed.

2. The display device according to Claim 1, wherein the intensity of the light having the wavelength is controlled based on time information.

3. The display device according to Claim 1, wherein the intensity of the light having the wavelength is controlled based on user instruction information set by a user.

4. The display device according to Claim 1, wherein the intensity of the light having the wavelength is controlled based on contents information indicating what type of program the image is.

5. The display device according to any one of Claims 1 to 4, comprising a complementary light emitter for emitting light whose color is substantially complementary to a color of the light having the wavelength.

6. The display device according to Claim 5, wherein a luminous intensity of the complementary light emitter is controlled in accordance with the intensity of the light having the wavelength.

7. A display device comprising an image display section for displaying an image, the image display section including pixels each of which has a plurality of light emitters, wherein:
the plurality of light emitters include a first light emitter for emitting light having such a wavelength that affects a biorhythm, and
a characteristic of a luminous intensity of the first light emitter with respect to a video signal inputted into the image display section is switched.

8. A display device comprising an image display section for displaying an image, the image display section including pixels each of which has a plurality of light emitters, wherein:
the plurality of light emitters include a first light emitter for emitting light having a dominant wavelength in a range of 445 nm to 480 nm, and
a characteristic of a luminous intensity of the first light emitter with respect to a video signal inputted into the image display section is switched.

9. The display device according to Claim 7 or 8, wherein the characteristic of the luminous intensity of the first light emitter with respect to the video signal is switched based on time information.

10. The display device according to Claim 7 or 8, wherein the characteristic of the luminous intensity of the first light emitter with respect to the video signal is switched based on user instruction information set by a user.

11. The display device according to Claim 7 or 8, wherein the characteristic of the luminous intensity of the first light emitter with respect to the video signal is switched based on contents information indicating what type of program the image is.

12. The display device according to any one of Claims 7 to 11, wherein the plurality of light emitters include a second light emitter for emitting red light and a third light emitter for emitting green light.

13. The display device according to any one of Claims 7 to 12, wherein the plurality of light emitters include a complementary light emitter for emitting light whose color is substantially complementary to a color of light emitted by the first light emitter.

14. The display device according to Claim 13, wherein a luminous intensity of the complementary light emitter is controlled in accordance with the luminous intensity of the first light emitter.

15. The display device according to Claim 13 or 14, wherein the complementary light emitter is disposed next to the first light emitter.

16. The display device according to any one of Claims 7 to 15, wherein at least one of the plurality of light emitters is a light-emitting diode.

17. The display device according to any one of Claims 7 to 15, wherein at least one of the plurality of light emitters is an electroluminescent light emitter.

18. A display device irradiating an image display section, which is for displaying an image, with light from a light source so as to cause the image display section to display the image, the display device comprising:
a plurality of light emitters which include a first light emitter for emitting light having such a wavelength that affects a biorhythm,
a luminous intensity of the first light emitter with respect to an video signal inputted into the image display section being switched.

19. A display device irradiating an image display section, which is for displaying an image, with light from a light source so as to cause the image display section to display the image, the display device comprising:
a plurality of light emitters which include a first light emitter for emitting light having a dominant wavelength in a range of 445 nm to 480 nm,
a luminous intensity of the first light emitter with respect to an video signal inputted into the image display section being switched.

20. The display device according to Claim 18 or 19, wherein the luminous intensity of the first light emitter is controlled based on time information.

21. The display device according to Claim 18 or 19, wherein the luminous intensity of the first light emitter is controlled based on user instruction information set by a user.

22. The display device according to Claim 18 or 19, wherein the characteristic of the luminous intensity of the first light emitter is controlled based on contents information indicating what type of program the image is.

23. The display device according to any one of Claims 18 to 22, wherein the light source includes a second light emitter for emitting red light and a third light emitter for emitting green light.

24. The display device according to any one of Claims 18 to 23, wherein the light source includes a white light emitter for emitting white light.

25. The display device according to any one of Claims 18 to 24, wherein the light source includes a complementary light emitter for emitting light whose color is substantially complementary to a color of light emitted by the first light emitter.

26. The display device according to Claim 25, wherein a luminous intensity of the complementary light emitter is controlled in accordance with the luminous intensity of the first light emitter.

27. The display device according to Claim 25 or 26, wherein the complementary light emitter is disposed next to the first light emitter.

28. The display device according to any one of Claims 18 to 24, comprising a fluorescent material for emitting light whose color is substantially complementary to a color of light emitted by the first light emitter.

29. The display device according to any one of Claims 18 to 28, wherein at least one of the light emitters of the light source is a light-emitting diode.

30. The display device according to any one of Claims 18 to 28, wherein at least one of the light emitters of the light source is an electroluminescent light emitter.
